# FASCICULE DE BREVET EUROPEEN

(11) **EP 2 524 229 B1**
(45) Date de publication et mention de la délivrance du brevet: **01.08.2018**
(21) Numéro de dépôt: 11705011.2
(22) Date de dépôt: 11.01.2011
(51) Int. Cl.: A61K 31/4188, C07D 495/04, C07D 333/06, C07D 233/22, C07D 207/444, G01N 33/543, G01N 33/569, G01N 33/535

(54) **PROCÉDÉ DE PRÉPARATION DE COMPOSÉS POLYBIOTINYLÉS**
VERFAHREN ZUR HERSTELLUNG VON POLYBIOTINYLIERTEN VERBINDUNGEN
METHOD FOR PREPARING POLYBIOTINYLATED COMPOUNDS

(30) Priorité: 12.01.2010 FR 1050174
(43) Date de publication de la demande: 21.11.2012
(73) Titulaire: bioMérieux, 69280 Marcy-l'Étoile (FR)
(72) Inventeur: LACOUX, Xavier, F-69380 Dommartin (FR)
(74) Mandataire: Murgitroyd & Company
(86) Numéro de dépôt international: PCT/FR2011/050048
(87) Numéro de publication internationale: WO 2011/086321

(56) Documents cités:
- WO-A1-03/072546
- WO-A2-00/72802
- WO-A2-2005/034909
- US-A1- 2006 228 325
- ULRIKA WESTERLIND ET AL: "Ligands of the asialoglycoprotein receptor for targeted gene delivery, part 1: Synthesis of and binding studies with biotinylated cluster glycosides containing N-acetylgalactosamine", GLYCOCONJUGATE JOURNAL, KLUWER ACADEMIC PUBLISHERS, BO LNKD- DOI:10.1023/B:GLYC.0000045095.86867.C0, vol. 21, no. 5, 1 janvier 2004 (2004-01-01), pages 227-241, XP019207015, ISSN: 1573-4986

## Description

La présente invention concerne le domaine du diagnostic. En particulier, elle concerne un procédé de production de composés dendrimères polybiotinylés utiles pour l'amplification de signal, notamment lors de la détection d'un analyte dans un test diagnostique.

Les sondes qui permettent la détection d'analytes tels que des protéines et des acides nucléiques sont largement utilisées et sont des outils de choix pour le diagnostic *in vitro* et *in vivo.* Toutefois, la détection de ces analytes peut être difficile car ils ne sont pas toujours présents en quantité suffisamment élevée. Un système d'amplification de la détection est donc nécessaire.

Afin de permettre la détection d'analytes en quantité insuffisante, plusieurs techniques ont été mises au point. Ces techniques consistent soit à augmenter la quantité d'analyte, comme par exemple avec les techniques de PCR ou NASBA, soit à amplifier la détection, comme par exemple en utilisant des structures à multiples marqueurs.

Ainsi, la demande de brevet EP0774119A décrit des conjugués à multiples marqueurs comprenant un support polymère ayant au maximum 100 unités monomères, qui contient 1 à 10 molécules d'haptène et 1 à 10 groupes marqueurs, ainsi que la préparation de ces conjugués par synthèse en phase solide (a) en introduisant, en des positions prédéterminées du support, des dérivés monomères qui sont couplés de manière covalente à des molécules d'haptène et/ou des groupes marqueurs et/ou (b) en couplant à des groupes réactifs du support, après la synthèse, des molécules d'haptène et/ou des groupes marqueurs.

Les dendrimères sont des macromolécules bien définies qui présentent de multiples embranchements et qui se terminent par de multiples groupements fonctionnels périphériques. Un des avantages des dendrimères, dû au fait qu'ils possèdent de multiples groupements fonctionnels, consiste en ce qu'ils peuvent être utilisés comme agents de couplage stables, comme décrit dans la demande de brevet WO03/072546 déposée par la Demanderesse. Un autre avantage réside aussi en ce que ce sont des structures à multiples marqueurs car leurs groupements fonctionnels périphériques peuvent être utilisés pour lier de multiples marqueurs tels que la biotine, les fluorophores ou leur combinaison. Ainsi, la demande de brevet WO01/02861 décrit des dendrimères
liés d'une part à 1 à 1200 sondes et d'autre part à 1 à 1200 marqueurs. Ces dendrimères sont utilisés pour l'amplification de signal.

D'autres dendrimères polybiotinylés ont également été décrits dans la demande de brevet WO00/72802.

Toutefois, les procédés conventionnels de préparation des dendrimères ont pour inconvénients que la synthèse des dendrimères est effectuée en phase liquide, ce qui est plus complexe et plus long qu'en phase solide. De plus, certains procédés produisent des dendrimères non neutres et non polarisés.

La Demanderesse a maintenant mis au point de façon inattendue un nouveau procédé de préparation de composés dendrimères polybiotinylés permettant de pallier les inconvénients des procédés de l'art antérieur en ce sens qu'il permet de préparer de façon reproductible et contrôlée des composés polybiotinylés qui sont :
- hydrosolubles bien qu'électriquement neutres,
- polarisés grâce à une distribution séparée et précise dans l'espace de leurs fonctions, dont la fonction biotine, et
- synthétisés en phase solide.

Ainsi, la présente invention a pour premier objet un procédé de préparation d'un composé de formule (I) : 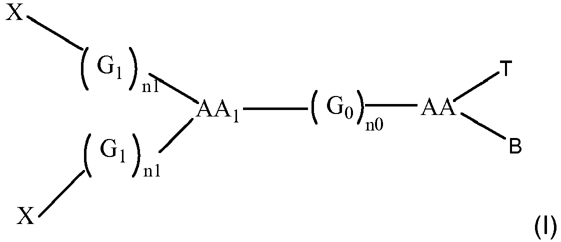 dans laquelle
- X est la biotine ou 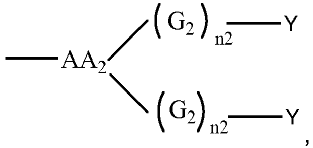
- Y est la biotine ou
- Y est la biotine ou 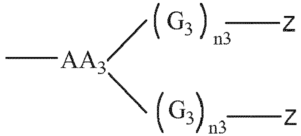
- Z est la biotine ou 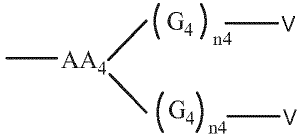
- V est la biotine ou 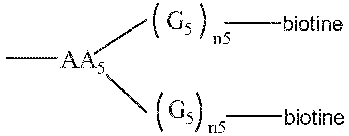
- B est NH₂ ou H,
- AA est un dérivé de molécule trifonctionnelle quand B est NH₂ ou de molécule difonctionnelle quand B est H,
- AA₁ à AA₅ sont chacun indépendamment un dérivé de molécule trifonctionnelle,
- G₀ à G₅ sont chacun indépendamment un bras comprenant au moins une unité (-CH₂-CH₂-O-),
- n₀ à n₅ sont chacun indépendamment un nombre entier compris entre 1 et 8 et
- T est un antiligand choisi parmi : haptène, anticorps, antigène, peptide, sucre, lectine, et polynucléotide ou un groupement réactif pour la fixation à un antiligand, ledit antiligand étant susceptible de réagir avec un ligand,
caractérisé en ce qu'il comprend les étapes consistant à :
(i) greffer n₀ composés de formule W"₀-G₀-OH, dans laquelle W"₀ est un groupement protecteur des amines, n₀ et G₀ étant tels que définis précédemment, à un composé de formule (II) : 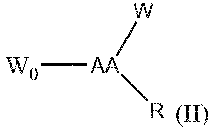 dans laquelle R est une résine préfonctionnalisée, W et W₀ sont différents l'un de l'autre et représentent un groupement protecteur des amines, W étant différent de W"₀, et AA est tel que défini précédemment,
   pour obtenir un composé de formule (III) : 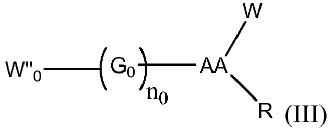
(ii) coupler un composé de formule (IV) : 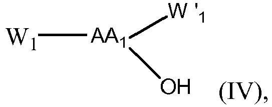 dans laquelle W₁ et W'₁ sont des groupements protecteurs des amines identiques ou différents l'un de l'autre et des groupements W₀ et W"₀, mais différents de W, et AA₁ est tel que défini précédemment, avec le composé de formule (III) pour obtenir un composé de formule (V) : 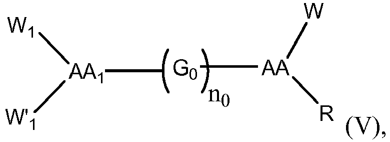
(iii) greffer 2n₁ composés de formule W"₁-G₁-OH, dans laquelle W"₁ est un groupement protecteur des amines, identique ou différent l'un de l'autre et des autres groupements protecteurs utilisés dans ce procédé, mais différents de W, G₁ et n₁ étant tels que définis précédemment, au composé obtenu dans l'étape (ii) pour obtenir un composé de formule (VI) : 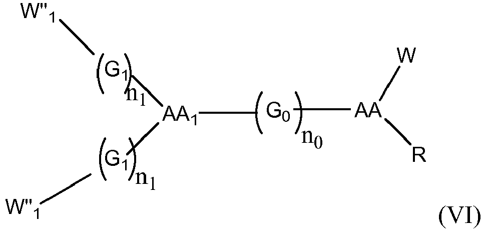
(iv) quand X de la formule (I) est la biotine, passer directement à l'étape (v) ; sinon, quand X de la formule (I) n'est pas la biotine, répéter les étapes (ii) et (iii) avec 2^{p-1} composés de formule (VII) : 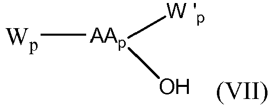 et 2^{p}nₚ fois le composé de formule W"ₚ-Gₚ-OH, dans laquelle p est un nombre entier compris entre 2 et 5, et Wₚ, W'ₚ, W"ₚ sont des groupements protecteurs des amines identiques ou différents les uns des autres et des autres groupements protecteurs utilisés dans ce procédé, mais différents de W, et AAₚ est une molécule trifonctionnelle,
   selon la séquence suivante :
   - 1 fois quand Y est la biotine, p étant alors égal à 2
   - 2 fois quand Y n'est pas la biotine et Z est la biotine, p étant alors égal à 2 puis à 3,
   - 3 fois quand Y et Z ne sont pas la biotine et V est la biotine, p étant alors égal à 2, 3 puis 4, et
   - 4 fois quand Y, Z et V ne sont pas la biotine, p étant alors égal à 2, 3, 4 puis 5,
(v) déprotéger le composé ainsi obtenu au niveau du groupement W"₁ ou W"ₚ, p étant compris entre 2 et 5, et coupler avec de la biotine,
(vi) déprotéger, au niveau du groupement W, le composé ainsi polybiotinylé et coupler avec un antiligand ou un groupement réactif pour la fixation à un antiligand (T) et
(vii) couper le composé ainsi obtenu de la résine (R) pour obtenir un composé de formule (I).

Un autre objet de l'invention concerne les composés de formule (I) dans laquelle :
- X est la biotine ou 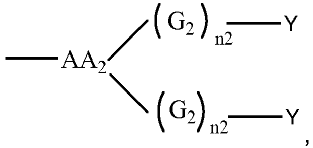
- Y est la biotine ou 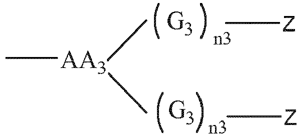
- Z est la biotine ou 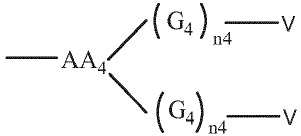
- V est la biotine ou 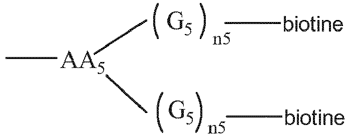
- B est NH₂ ou H,
- AA est un dérivé de molécule trifonctionnelle quand B est NH₂ ou un dérivé de molécule difonctionnelle quand B est H,
- AA₁ à AA₄ sont chacun indépendamment un dérivé de molécule trifonctionnelle,
- G₀ à G₅ sont chacun indépendamment un bras comprenant une unité (-CH₂-CH₂-O-),
- n₀ à n₅ sont chacun indépendamment un nombre entier compris entre 1 et 8 et
- T est un groupement maléimide, un groupement acide carboxylique ou un antiligand choisi parmi : haptène, anticorps, antigène, peptide, sucre, lectine, et polynucléotide ;
ladite molécule trifonctionnelle comportant trois fonctions identiques ou différentes choisies parmi NH₂ et COOH.

L'invention concerne enfin l'utilisation des composés susceptibles d'être obtenus par le procédé décrit ci-dessus, pour l'amplification de signal dans un test diagnostique.

Le procédé de l'invention permet donc de préparer des composés polybiotinylés de façon reproductible et contrôlée. Par ailleurs, lesdits composés obtenus sont très solubles même sans charge électrique du fait de la présence des substituants G₀ à G₅. Leur structure dendrimère particulière permet une polarisation de la molécule, dont l'encombrement stérique est contrôlé, ce qui permet une meilleure présentation des biotines et donc l'amplification du signal, notamment lorsqu'elle est utilisée dans une application diagnostique, du fait de la présence de 2 à 32 biotines. Les composés obtenus sont alors particulièrement utiles lors de la détection d'un analyte en très faible quantité.

La structure dendrimère des composés obtenus avec le procédé de l'invention est obtenue grâce à la mise en oeuvre de dérivés de molécules trifonctionnelles ou difonctionnelles.

Par molécule difonctionnelle, on entend toute molécule comportant au moins un atome trivalent ou tétravalent portant deux fonctions leur permettant de réagir avec deux molécules. Ces fonctions sont choisies parmi NH₂ et COOH. Ces molécules difonctionnelles peuvent alors comporter deux fonctions identiques ou différentes, à savoir deux fonctions NH₂, deux fonctions COOH ou bien une fonction COOH et une fonction NH₂. A titre d'exemple non limitatif de molécules difonctionnelles, on peut citer les diamines difonctionellles telles que l'éthylènediamine et la 4,7,10-trioxatridécanediamine.

Par dérivé de molécule difonctionnelle, on entend la molécule difonctionnelle dans laquelle chacune des deux fonctions est en engagement de liaison avec une autre entité chimique. Ainsi, le dérivé de molécule difonctionnelle est constitué du squelette de la molécule difonctionnelle dans laquelle chaque fonction NH₂ a perdu un atome d'hydrogène et chaque fonction COOH a perdu un radical hydroxyle (OH). Ainsi, par exemple, lorsque le dérivé de molécule difonctionnelle est l'éthylènediamine, de formule H₂N-CH₂-CH₂-NH₂, le dérivé d'éthylènediamine est -HN-CH₂-CH₂-NH-.

Lorsque le substituant AA est un dérivé de molécule difonctionnelle, B est H dans la formule (I).

Par molécule trifonctionnelle, on entend toute molécule comportant au moins un atome trivalent ou tétravalent portant trois fonctions leur permettant de réagir avec trois molécules. Ces fonctions sont choisies parmi NH₂ et COOH. Ces molécules trifonctionnelles peuvent alors comporter trois fonctions identiques ou différentes, à savoir trois fonctions NH₂, deux fonctions NH₂ et une fonction COOH, deux fonctions COOH et une fonction NH₂ ou bien trois fonctions COOH. A titre d'exemple non limitatif de molécules trifonctionnelles, on peut citer la lysine, l'acide diaminobutanoïque, l'acide diaminopropanoïque, la L-ornithine et leurs dérivés.

Par dérivé de molécule trifonctionnelle, on entend la molécule trifonctionnelle dans laquelle chacune des trois fonctions est en engagement de liaison avec une autre entité chimique. Ainsi, le dérivé de molécule trifonctionnelle est constitué du squelette de la molécule trifonctionnelle dans laquelle chaque fonction NH₂ a perdu un atome d'hydrogène et chaque fonction COOH a perdu un radical hydroxyle (OH). Ainsi, par exemple, lorsque le dérivé de molécule trifonctionnelle est la lysine, de formule H₂N-(CH₂)₄-CH(NH₂)-COOH, le dérivé de lysine est -HN -(CH₂)₄-CH(NH-)-CO-.

Lorsque le substituant AA est un dérivé de molécule trifonctionnelle, B est NH₂ dans la formule (I).

Selon un mode de réalisation préféré, les composés dans le procédé de l'invention présentent l'une des caractéristiques suivantes :
- les substituants AA₁ et AA₂, le cas échéant AA₃, AA₄ et AA₅, sont identiques et sont de préférence un dérivé de lysine,
- le substituant AA est un dérivé de lysine et B est NH₂.

La solubilité des composés obtenus avec le procédé de l'invention provient de la nature des bras espaceurs G₀ à G₅ utilisés car ils comprennent au moins une unité (-CH₂-CH₂-O-). De façon préférée, les composés de l'invention comprennent de une à six unités (-CH₂-CH₂-O-), de préférence encore de une à quatre unités (-CH₂-CH₂-O-). A titre d'exemples non limitatifs de tels bras espaceurs, on peut citer le pentaoxaoctadécanoyle, le tétraoxapentadécanoyle, le trioxadodécanoyle, le trioxatridécanoyle, le dioxaoctanoyle, l'oxapentoyle et l'héxaoxahénéicosanoyle et leurs dérivés.

Selon un mode de réalisation, les composés dans le procédé de l'invention présentent l'une des caractéristiques suivantes :
- les substituants G₀, G₁, le cas échéant G₂, G₃, G₄ et G₅, sont identiques et sont de préférence de formule (-NH-CH₂-CH₂-O-CH₂-CH₂-O-CH₂-CO-).
- les nombres entiers n₀, n₁, le cas échéant n₂, n₃, n₄ et n₅, sont identiques et sont de préférence égaux à 2 ou 3.

Dans les composés préparés selon le procédé de l'invention, T est un antiligand ou un groupement réactif pour la fixation à un antiligand.

Par antiligand, on entend toute molécule capable de se lier avec un ligand. Les exemples de couples ligand/anti-ligand sont bien connus de l'homme du métier, ce qui est le cas par exemple des couples suivants : haptène/anticorps, antigène/anticorps, peptide/anticorps, sucre/lectine, polynucléotide/complémentaire du polynucléotide.

Les groupements réactifs pour la fixation à un antiligand sont largement connus de l'homme du métier. Des exemples non limitatifs de tels groupements sont les groupements maléimide, acide carboxylique, thiol, amine, alcoxyamine, hydrazine, azido, alcyne, aldehyde.

Les composés obtenus par le procédé de l'invention permettent l'amplification du signal de détection dans un test diagnostic du fait de la présence de plusieurs biotines, selon le tableau 1 suivant :

**Tableau 1**

| Nombre de biotines | X | Y | Z | V |
|---|---|---|---|---|
| 2 | biotine | NA | NA | NA |
| 4 | 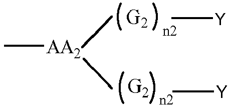 | biotine | NA | NA |
| 8 | 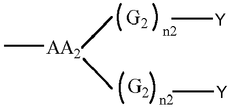 | 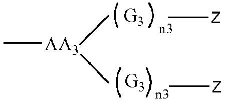 | biotine | NA |
| 16 | 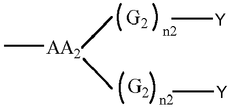 | 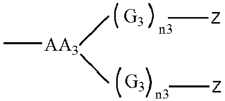 | 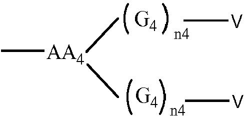 | biotine |

| | | | | |
|---|---|---|---|---|
| NA: non applicable | | | | |

Selon un mode de réalisation particulier de l'invention, X est 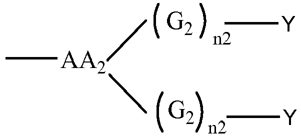 et Y est la biotine.

Les étapes de greffage et de couplage du procédé de l'invention sont des étapes classiquement utilisées par l'homme du métier dans le domaine de la synthèse en phase solide de type synthèse peptidique. Ces étapes peuvent être mises en oeuvre de façon manuelle ou bien de façon automatisée sur les synthétiseurs du commerce tels que le synthétiseur ABI 431 A et ABI433A.

Les réactifs utilisés lors de ces étapes sont connus de l'homme du métier et sont décrits par exemple dans Chemical Approaches to the Synthesis of Peptides & Proteins, Paul Lloyd Williams, Fernando Albericio, Ernest Giralt, CRC Press.

Ainsi, les composés de formule (II) dans laquelle R est une résine préfonctionnalisée et AA est un dérivé de molécule difonctionnelle sont disponibles chez Novabiochem. A titre d'exemple, on peut citer :
- le composé Universal NovaTag resin, référence 04-12-3910 de formule : 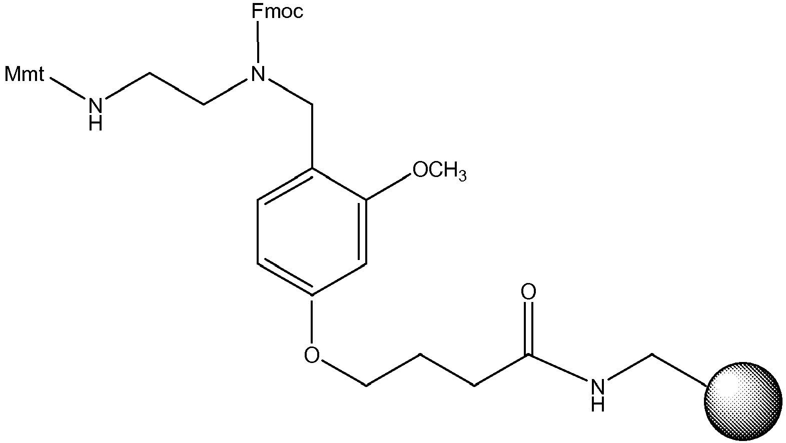 et
- le composé Universal PEG NovaTag resin, référence 04-12-3911 de formule :

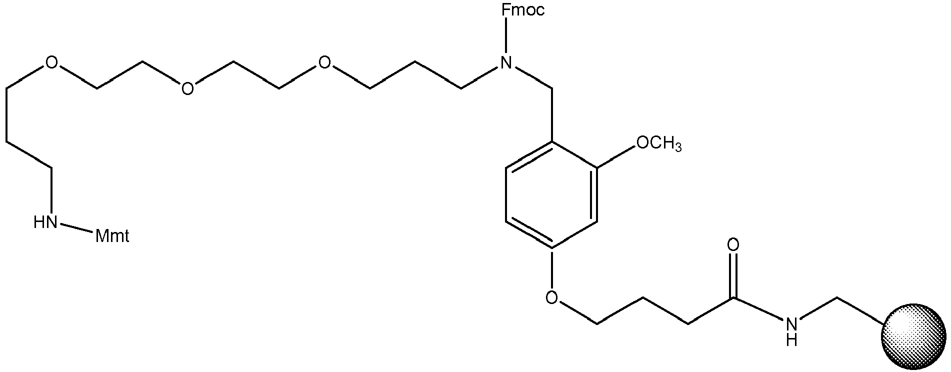

Lorsque AA est une molécule trifonctionnelle dans les composés de formule (II), ces derniers peuvent être obtenus en couplant un composé de formule (II') 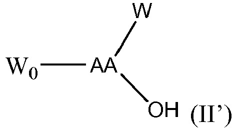 dans laquelle Wₒ et W sont tels que définis précédemment, sur une phase solide aminée de formule (II") Wᵣ-R dans laquelle R est 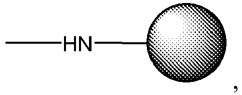 et Wᵣ est un groupement protecteur des amines identique ou différent de Wₒ et W.

Un exemple de phase solide aminée comprend la résine RINK-amide-MHBA de Novabiochem (Référence 01-64-0037).

Les étapes de déprotection et de séparation du composé polybiotinylé de la résine R sont largement connues de l'homme du métier et sont décrits par exemple dans Chemical Approaches to the Synthesis of Peptides & Proteins (*supra*).

Les groupements protecteurs des amines sont également largement connus de l'homme du métier et on peut citer, à titre d'exemples non limitatifs, le t-butyloxycarbonyle (Boc), le benzyloxycarbonyle, le 9-fluorénylméthoxycarbonyle (Fmoc) ou le méthoxytrityle (Mmt). On peut mettre en oeuvre n'importe quel groupement protecteur dans le procédé de l'invention dans la mesure où W est différent des autres groupements protecteurs utilisés.

Selon un mode de réalisation, les composés dans le procédé de l'invention présentent l'une des caractéristiques suivantes :
- le groupement W est le méthoxytrityle,
- les groupements W₁ et W'₁ sont identiques et, le cas échéant, W₂ et W'₂ sont identiques, W₃ et W'₃ sont identiques, W₄ et W'₄ sont identiques et W₅ et W'₅ sont identiques,
- les groupements W₁, W'₁ et, le cas échéant, W₂, W'₂, W₃, W'₃, W₄ et W'₄, W₅ et W'₅ sont des groupements fluorénylméthoxycarbonyle.

Le procédé de l'invention permet de préparer de nouveaux composés dendrimères polybiotinylés de formule (I) 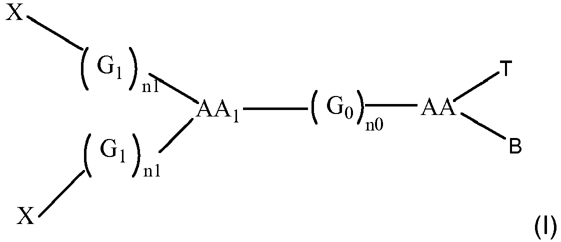 dans laquelle
- X est la biotine ou 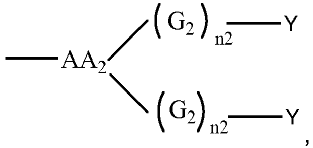
- Y est la biotine ou 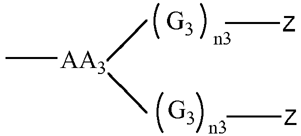
- Z est la biotine ou 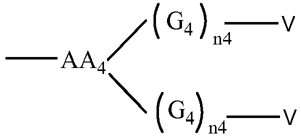
- V est la biotine ou 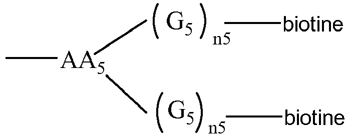
- B est NH₂ ou H,
- AA est un dérivé de molécule trifonctionnelle quand B est NH₂ ou un dérivé de molécule difonctionnelle quand B est H,
- AA₁ à AA₄ sont chacun indépendamment un dérivé de molécule trifonctionnelle,
- G₀ à G₅ sont chacun indépendamment un bras comprenant une unité (-CH₂-CH₂-O-),
- n₀ à n₅ sont chacun indépendamment un nombre entier compris entre 1 et 8 et
- T est un groupement maléimide, un groupement acide carboxylique ou un antiligand choisi parmi : haptène, anticorps, antigène, peptide, sucre, lectine, et polynucléotide ; ladite molécule trifonctionnelle comportant trois fonctions identiques ou différentes choisies parmi NH₂ et COOH.

Selon un mode de réalisation particulier, les composés de l'invention comprennent l'une des caractéristiques suivantes :
- les substituants AA₁ et AA₂, le cas échéant AA₃, AA₄ et AA₅, sont identiques et sont de préférence un dérivé de lysine,
- les substituants G₀, G₁, le cas échéant G₂, G₃, G₄ et G₅, sont identiques et sont de préférence de formule (-NH-CH₂-CH₂-O-CH₂-CH₂-O-CH₂-CO-),
- les nombres entiers n₀, n₁, le cas échéant n₂, n₃, n₄ et n₅, sont identiques et sont de préférence égaux à 2 ou 3,
- X est 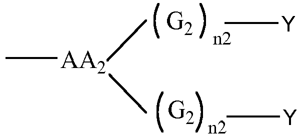 et Y est la biotine,
- AA est un dérivé de lysine et B est NH₂,
- l'antiligand est un fragment Fab',
- X est 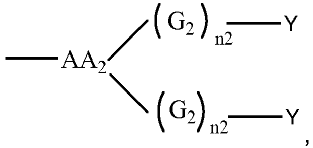
Y est la biotine, AA, AA₁ et AA₂ sont un dérivé de lysine, G₀, G₁ et G₂ sont de formule (-NH-CH₂-CH₂-O-CH₂-CH₂-O-CH₂-CO-), n₀, n₁ et n₂ sont égaux à 2, B est NH₂ et T est un antiligand, de préférence un fragment Fab'.

Les composés préparés par le procédé de l'invention sont particulièrement utiles pour l'amplification de signal dans les méthodes de diagnostic *in vitro* impliquant une reconnaissance d'un couple ligand/anti-ligand, la molécule à diagnostiquer constituant le ligand, ce qui constitue un autre objet de l'invention.

En effet, les composés de l'invention possèdent directement un anti-ligand (substituant T) ou sont mis à réagir avec un antiligand qui se fixera au niveau dudit substituant T par des méthodes largement connues de l'homme du métier.

Les exemples de couples ligand/anti-ligand sont bien connus de l'homme du métier, ce qui est le cas par exemple des couples suivants : haptène/anticorps, antigène/anticorps, peptide/anticorps, sucre/lectine, polynucléotide/complémentaire du polynucléotide.

Les méthodes diagnostic *in vitro* pouvant mettre en oeuvre les conjugués de l'invention sont notamment les tests immunologiques tels que les méthodes « sandwich » telles qu'ELISA, IRMA et RIA, les méthodes dites de compétition et les méthodes d'immunodétection directe comme l'immunohistochimie, l'immunocytochimie, le Western-blot et le Dot-blot. Toutes ces méthodes sont largement connues de l'homme du métier.

Les méthodes de diagnostic mettant en oeuvre les composés préparés selon le procédé de l'invention sont utiles tant dans le diagnostic clinique de maladie que dans le diagnostic de produits préparés en Industrie (diagnostic industriel) tels que les produits destinés à l'agroalimentaire ou les produits destinés à la santé.

Les maladies pouvant être diagnostiquées avec les composés de l'invention ne sont pas limitées et comprennent toutes maladies révélées par la présence d'un marqueur spécifique de la maladie, du type molécule d'intérêt biologique ou analyte ou ligand, pour lequel il existe un partenaire de liaison. A titre d'exemples non limitatifs, on peut citer les maladies virales telles les hépatites et le SIDA, et les cancers solides telles que le cancer du sein, du colon ou de la prostate.

L'amplification de signal est mise en oeuvre par l'utilisation d'une molécule marqueur capable de générer directement ou indirectement un signal détectable. Une liste non limitative de ces marqueurs consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que les Alexa ou les phycocyanines,
étant entendu que ces marqueurs seront modifiés pour être lié à un partenaire de liaison à la biotine tel que la streptavidine. Ce type de modification est largement connu de l'homme du métier.

Selon le type de marquage utilisé, l'homme du métier ajoutera des réactifs permettant la visualisation du marquage.

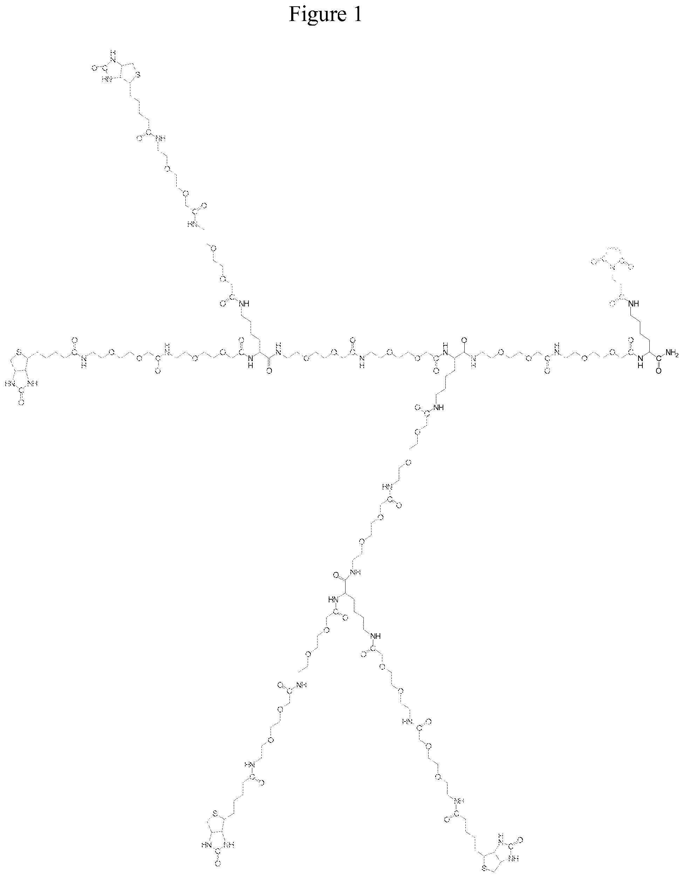

L'invention sera mieux comprise à l'aide des exemples suivants qui sont donnés à titre illustratif et non limitatif, ainsi qu'à l'aide des figures 1 à 8, dans lesquelles :
- la figure 1 correspond à la formule développée d'un composé à 4 biotines selon un mode de réalisation,

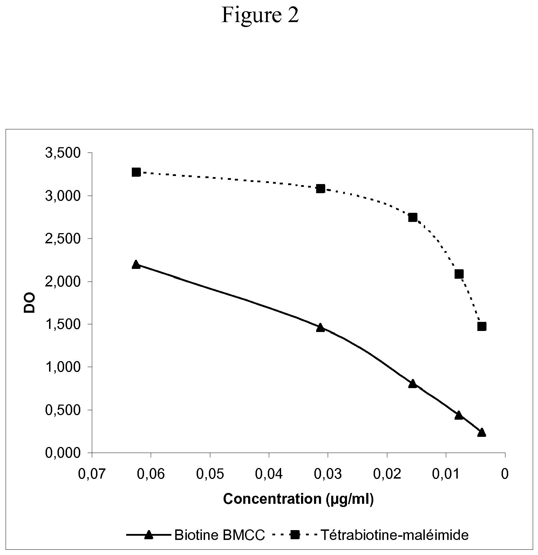

- la figure 2 est une représentation graphique donnant les résultats de DO d'un test ELISA direct d'un conjugué Fab' anti-*Listeria monocytogenes* monobiotinylé (par de la biotine BMCC) ou tétrabiotinylé (par un composé selon un mode de réalisation de l'invention), fixé au fond d'une microplaque, en fonction de sa concentration,

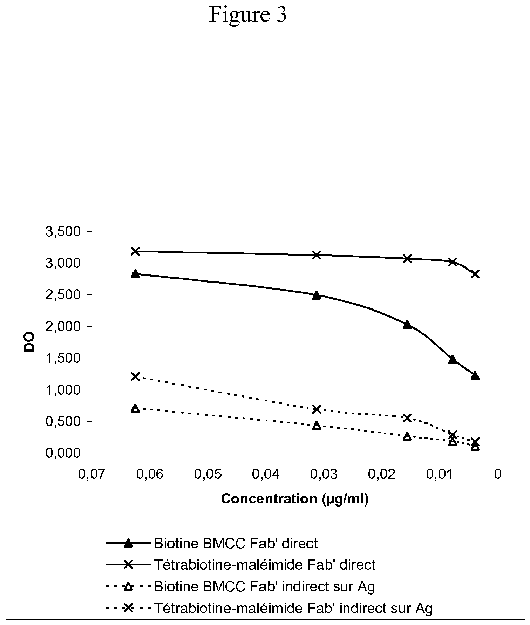

- la figure 3 est une représentation graphique donnant les résultats de DO d'un test ELISA direct d'un conjugué Fab' anti-Salmonelle monobiotinylé (par de la biotine BMCC) ou tétrabiotinylé (par un composé selon un mode de réalisation de l'invention), fixés au fond d'une microplaque, en fonction de leur concentration, ainsi que d'un test ELISA indirect avec ces mêmes conjugués, mais détectés par l'intermédiaire d'antigènes de Salmonelle,

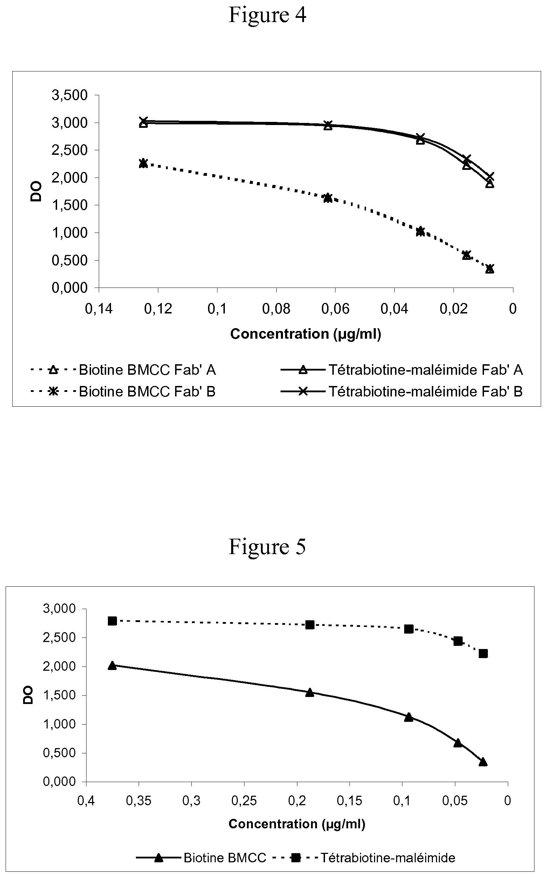

- la figure 4 est une représentation graphique donnant les résultats de DO d'un test ELISA direct d'un conjugué Fab' anti-HIV monobiotinylé (par de la biotine BMCC) ou tétrabiotinylé (par un composé selon un mode de réalisation de l'invention), fixé au fond d'une microplaque, en fonction de sa concentration,
- la figure 5 est une représentation graphique donnant les résultats de DO d'un test ELISA direct d'un conjugué Fab' anti-Kallikréine monobiotinylé (par de la biotine BMCC) ou tétrabiotinylé (par un composé selon un mode de réalisation de l'invention), fixé au fond d'une microplaque, en fonction de sa concentration,

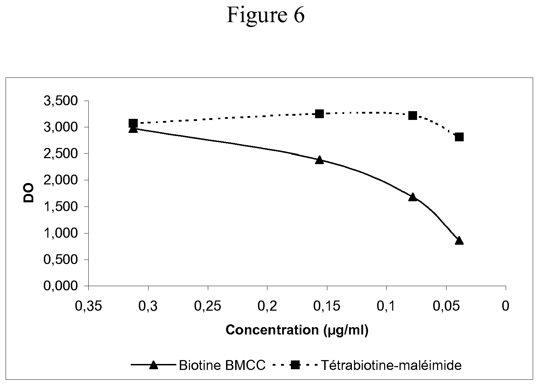

- la figure 6 est une représentation graphique donnant les résultats de DO d'un test ELISA direct d'un conjugué antigène gp160 réduit au DTT monobiotinylé (par de la biotine BMCC) ou tétrabiotinylé (par un composé selon un mode de réalisation de l'invention), fixé au fond d'une microplaque, en fonction de sa concentration,

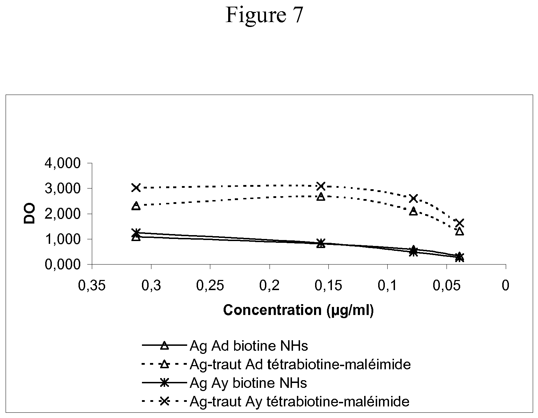

- la figure 7 est une représentation graphique donnant les résultats de DO d'un test ELISA direct de conjugués antigènes de surface de l'hépatite B monobiotinylés (par de la biotine NHs) ou tétrabiotinylés (par un composé selon un mode de réalisation de l'invention), fixés au fond d'une microplaque, en fonction de leur concentration, et

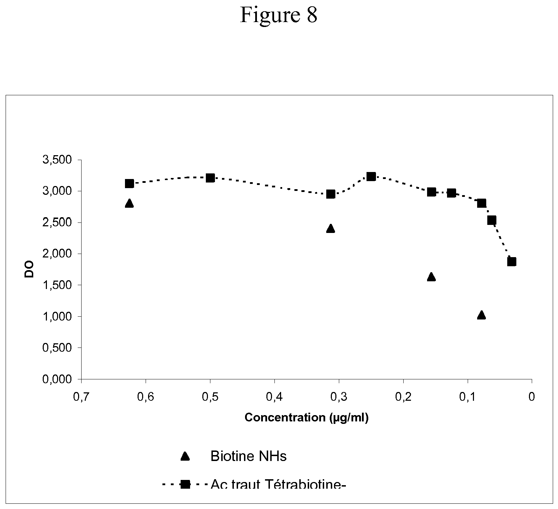

- la figure 8 est une représentation graphique donnant les résultats de DO d'un test ELISA direct d'un conjugué anticorps anti-Salmonelle monobiotinylé (par de la biotine NHs) ou tétrabiotinylé (par un composé selon un mode de réalisation de l'invention), fixé au fond d'une microplaque, en fonction de sa concentration.

### Exemple 1 : Préparation d'un composé de formule (I) présentant 4 biotines

(AA=AA₁=AA₂= dérivé de lysine ;
B est NH₂ ;
G₀=G₁=G₂= (-NH-CH₂-CH₂-O-CH₂-CH₂-O-CH₂-CO-) = Ado;
n₀=n₁=n₂= 2
T= maléimide
X= 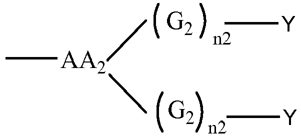
Y = biotine)

On a disposé dans un réacteur du synthétiseur automatique 433A (Applied Biosystems) équipé d'un détecteur UV, environ 132 µmoles de la résine Fmoc-RINK-MBHA (Novabiochem). Par ailleurs, on a préparé les cartouches contenant chacun des synthons à raison de 1 mmole par cartouche :
- Fmoc-Lys(Mmt)-OH, 640,8 mg (Novabiochem)
- Fmoc-Lys(Fmoc)-OH, 590,7 mg (Novabiochem)
- Fmoc-Ado-OH, 385,4 mg (ou Fmoc-8-amino-3,6-dioxaoctanoic acid, Polypeptide)
- N-maleoyl-βalanine, 169,1 mg (FLUKA)
- Biotine, MW 244,3 (SIGMA) préalablement reprise en solution à 0,5 M dans le DMSO (ALDRICH), soit 2 ml de solution par cartouche

On a utilisé les matières premières suivantes :
- N-méthyl-pyrrolidone ou NMP (Applied Biosystems)
- Dichlorométhane ou DCM (Applied biosystems)
- Pipéridine (ALDRICH)
- Anhydride acétique (FLUKA)
- Méthanol (MERCK)
- Solution de di-isopropyléthylamine ou DIEA (ALDRICH), 2 M dans la NMP ci-dessus
- Solution de hexafluorophosphate benzotriazolyl tétraméthyluronium ou HBTU (Novabiochem) à l'équivalence avec du N-hydroxybenzotriazole (SENN) le tout en solution à environ 0,45 M dans du N,N-diméthylformamide ou DMF (ALDRICH)
- Solution à 1% d'acide trifluoroacétique ou TFA (ACROS) et à 2,5% de tri-isopropylsilane ou TIS (FLUKA) dans le même DCM que ci-dessus.

Par défaut, on a prévu 2 cartouches de chaque synthon à incorporer de manière à travailler en mode « Conditional Double Coupling ».

La programmation du synthétiseur repose sur une succession de cycles, eux-mêmes divisés en modules, les modules sont une suite de fonctions primaires sur l'automate. On a utilisé comme base les programmes du fournisseur qui ont été adaptés pour la réalisation de l'exemple suivant :
Les modules utilisés sont les suivant :
- A : reprise de la cartouche d'acide aminé en poudre
- B : déprotection du groupement Fmoc N-terminal à la pipéridine
- C : *capping* ou masquage à l'anhydride acétique des amines n'ayant pas réagi
- D : rinçages de la résine à la NMP
- E : activation et transfert de la solution d'acide aminé vers la résine
- F : couplage sous agitation
- G : déprotection du monométhoxytrityle de la chaîne latérale de la 1^{ère} lysine
- I : agitation intermittente du réacteur pendant 30 minutes
- a : module conditionnel de reprise de la cartouche, rinçage de la résine et activation / transfert vers le réacteur
- b : module conditionnel de déprotections supplémentaires à la pipéridine
- c : rinçage de la résine au DCM
- d : autre module de rinçage de la résine à la NMP
- f : module conditionnel de couplage long sous agitation
- i : module conditionnel d'éjection de cartouche non utilisée
On a programmé le synthétiseur selon les cycles tels qu'indiqués dans le tableau 2 suivant :

**Tableau 2**

| **Cycle** | **AA** | **Nom** | **Modules** |
|---|---|---|---|
| 1 | Fmoc-Lys(Mmt)-OH | Double couplage conditionnel et capping | BbADEFfiafCd |
| 2 | Fmoc-Ado-OH | Double couplage conditionnel et capping | BbADEFfiafCd |
| 3 | Fmoc-Ado-OH | Double couplage conditionnel et capping | BbADEFfiafCd |
| 4 | Fmoc-Lys(Fmoc)-OH | Double couplage conditionnel et capping | BbADEFfiafCd |
| 5 | Fmoc-Ado-OH | Double couplage conditionnel long et capping | BbADEFfIiafICd |
| 6 | Fmoc-Ado-OH | Double couplage conditionnel long et capping | BbADEFfIiafICd |
| 7 | Fmoc-Lys(Fmoc)-OH | Double couplage conditionnel long et capping | BbADEFfIiafICd |
| 8 | Fmoc-Ado-OH | Double couplage conditionnel très long et capping | BbADEFfIIiafIICd |
| 9 | Fmoc-Ado-OH | Double couplage conditionnel très long et capping | BbADEFfIIiafIICd |
| 10 | Biotine | Double couplage conditionnel très long et capping | BbADEFfIIiafIICd |
| 11 | N-maleoyl-βalanine | Déprotection Lys(Mmt) double couplage long et capping | GcADEFIAdEFfICd |
| 12 | Aucun | Lavages résine NMP et DCM | Dcc |

Lors des déprotections du groupement Fmoc, l'appareil effectue un test. En fonction du résultat obtenu, le programme effectue un simple ou double couplage (utilise respectivement 1 seule ou bien les 2 cartouches prévues)

### 1.1. Couplage 1 ^{ère} lysine (AA)

On déprotège la résine et on couple la Fmoc-Lys(Mmt)-OH (II') comme suit (cycle 1 : l'appareil utilise 1 seule ou les 2 cartouches de l'acide aminé Fmoc-Lys(Mmt)-OH en fonction du résultat du test conditionnel) : 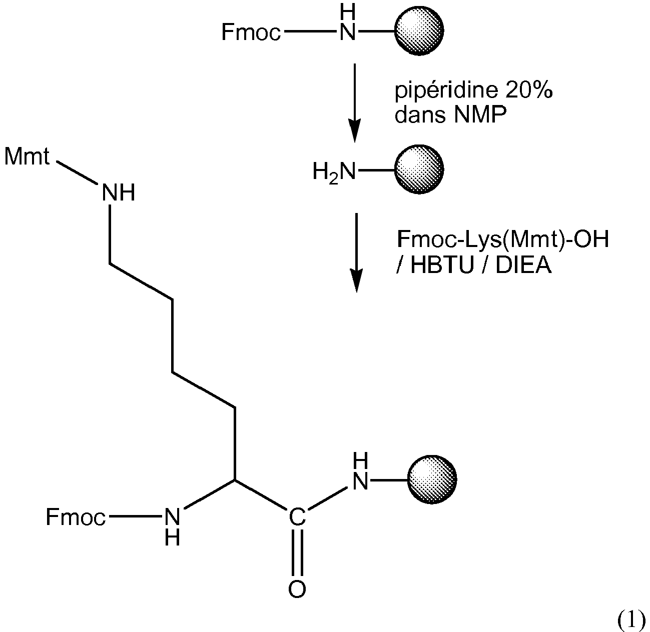 (1) ou (II") : également représenté par

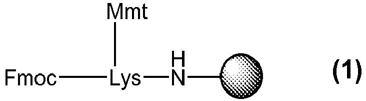

### 1.2. Construction du squelette :

Il consiste en l'alternance de :
a) greffage de bras espaceur hydrophile Ado₂
b) couplage du synthon de ramification = Fmoc-Lys(Fmoc)-OH

### A partir de (1) ci-dessus

### i) greffage de bras espaceur hydrophile Ado₂ (étape a)

(cycles 2 et 3 : l'appareil utilise 1 seule ou les 2 cartouches de l'acide aminé Fmoc-Ado-OH à chaque cycle en fonction du résultat des tests conditionnels)

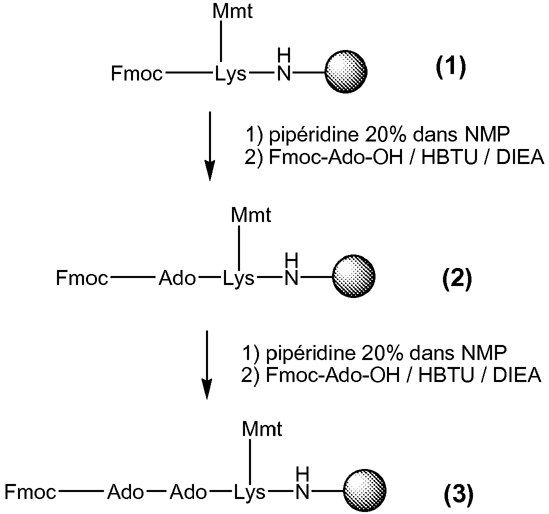

### ii) Ramification par Fmoc-Lys(Fmoc)-OH (étape b)

(cycle 4 : l'appareil utilise 1 seule ou les 2 cartouches de l'acide aminé Fmoc-Lys(Fmoc)-OH en fonction du résultat du test conditionnel)

### A partir de (3)

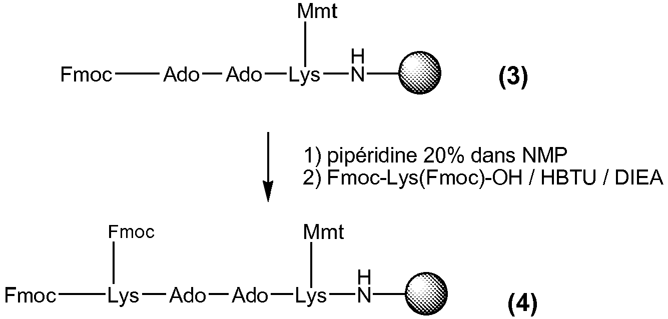

### iii) Répétition des étapes a), b) puis a pour terminer le squelette

- cycles 5 et 6 : l'appareil utilise 1 seule ou les 2 cartouches à chaque acide aminé Fmoc-Ado-OH en fonction du résultat des tests conditionnels : 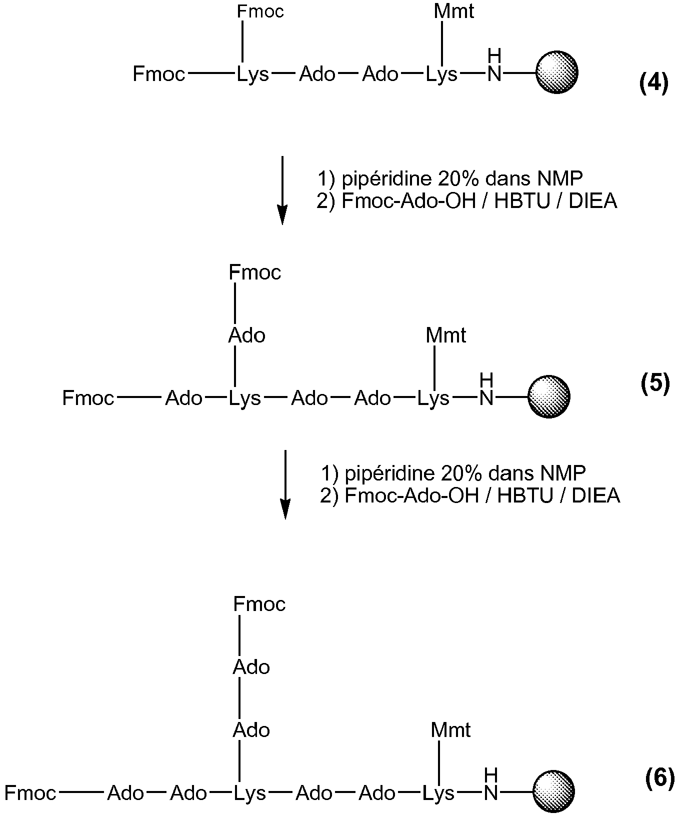
- cycle 7 : l'appareil utilise 1 seule ou les 2 cartouches de l'acide aminé Fmoc-Lys(Fmoc)-OH en fonction du résultat du test conditionnel : 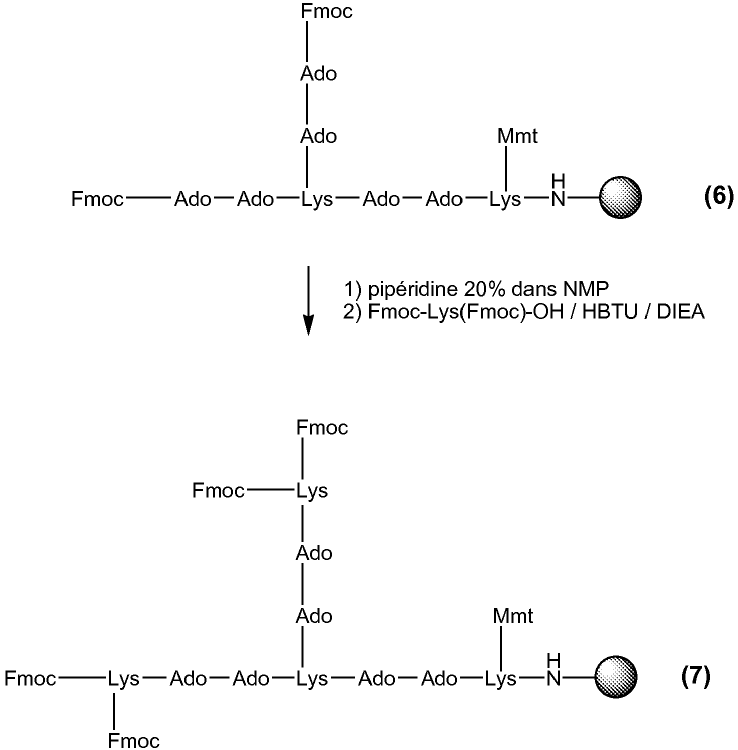
- cycles 8 et 9 : l'appareil utilise 1 seule ou les 2 cartouches de l'acide aminé Fmoc-Ado-OH en fonction du résultat des tests conditionnels :

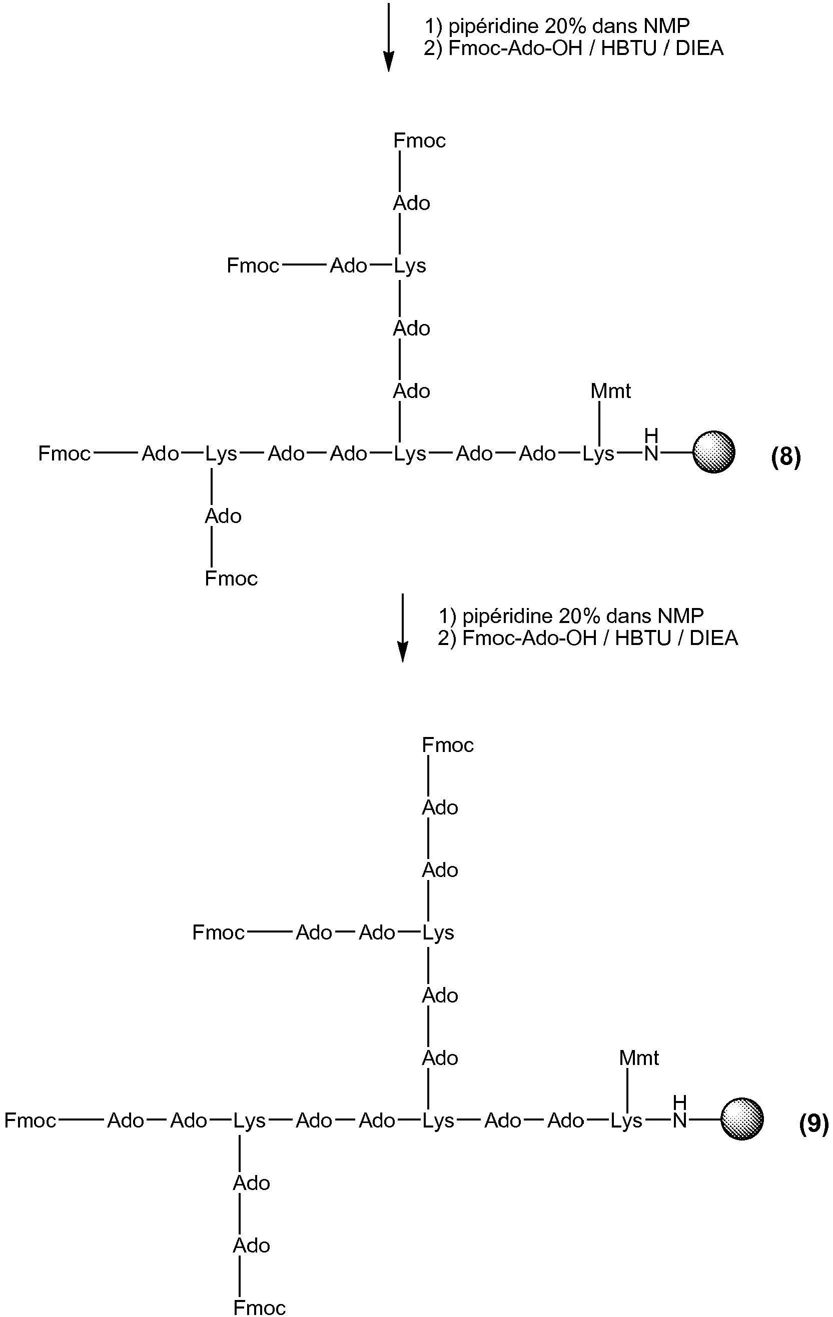

### 1.3. Couplage d'une biotine à chaque extrémité des 4 branches :

(cycle 10 : l'appareil utilise 1 seule ou les 2 cartouches de biotine selon le résultat du test conditionnel)

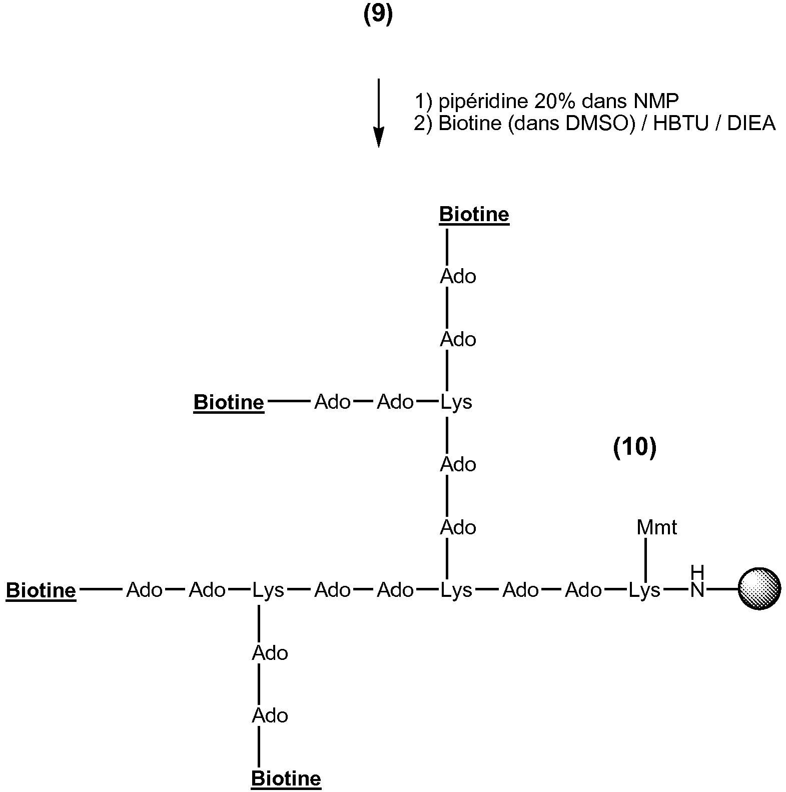

### 1.4. Déprotection du Mmt et greffage du maléimide :

(cycle 11 : cette fois, le programme utilise les 2 cartouches de N-maleoyl-βalanine - Pas de test conditionnel).

Le groupe protecteur Mmt est coupé sélectivement par action répétée d'une solution de DCM (dichlorométhane) à 1.5% de TFA (acide trifluoroacétique) et 1.5% de TIS (tri-isopropylsilane). L'amine de la chaîne latérale de la lysine insérée à l'étape 1) est ainsi libérée pour son couplage avec une forme acide de maléimide, N-maleoyl-β-alanine. soit :

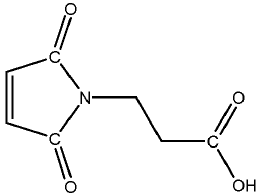

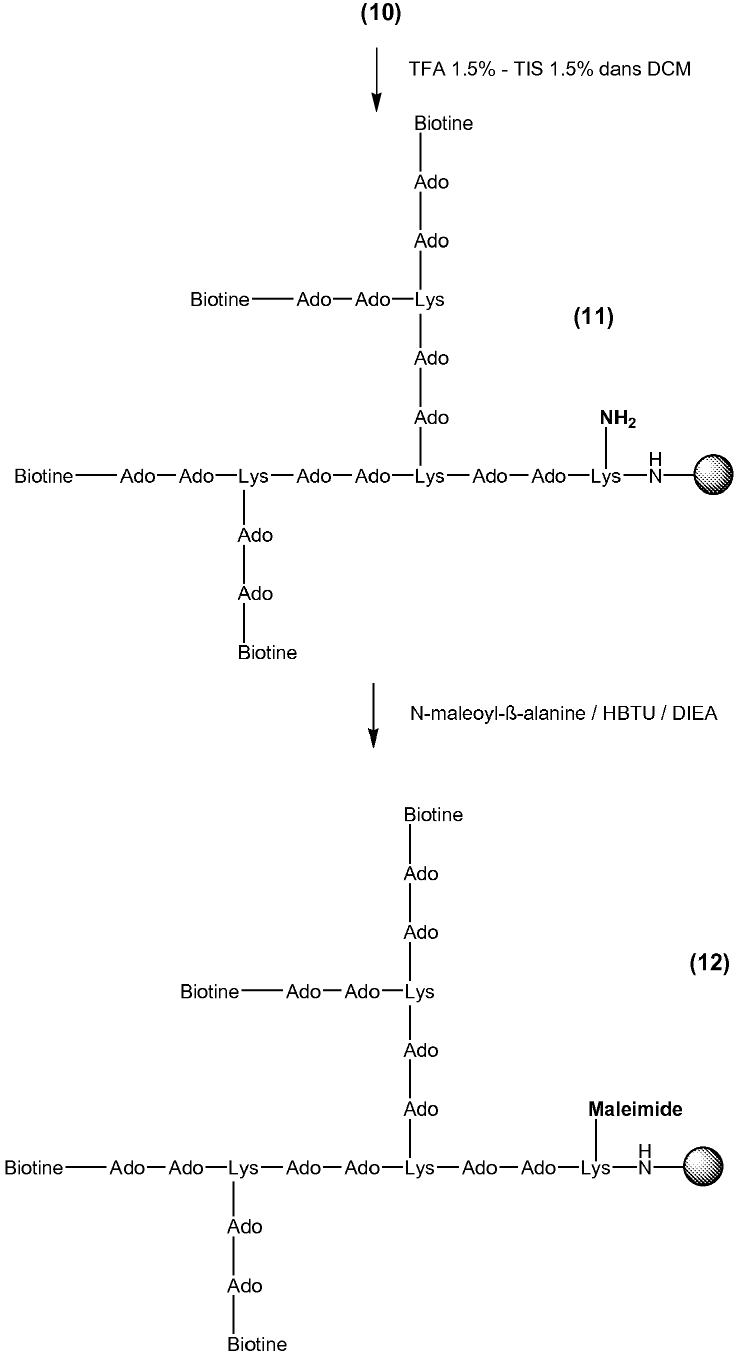

### 1.5. Clivage

Après synthèse, la résine est séchée sous courant d'azote. Elle est transférée dans une seringue plastique de 20 ml comportant un fritté et mise en contact avec 10 ml d'une solution de TFA/eau (95/5) sous agitation pendant 1h30 à température ambiante. Après quoi la solution de clivage est collectée par filtration (via le fritté de la seringue) dans un ballon en verre. La résine est rincée par environ 5 ml de TFA, puis par 3 fois environ 5 ml de DCM. Tous ces volumes sont rajoutés au ballon. Le mélange brut de clivage est évaporé sous pression réduite avec un évaporateur rotatif dont le bain est à température ambiante pendant quelques dizaines de minutes. On obtient au final un résidu huileux de couleur orangée.

Ce produit est repris dans quelques millilitres d'eau déminéralisée pour analyses et purification.

Une aliquote rediluée dans l'eau déminéralisée est analysée par HPLC et spectrométrie de masse couplée à l'HPLC.

HPLC analytique BECKMAN avec colonne de chromatographie phase inverse C₁₈ VYDAC de 5,4 mm de diamètre par 250 mm de long, avec les éluants A = eau à 0,1% de TFA et B = mélange acétonitrile ou ACN avec eau (respectivement 95/5) à 0,1% de TFA. Le débit est de 1 ml/min. A partir de l'injection de l'échantillon, on procède à un gradient de 0 à 80% d'acétonitrile en 30 minutes. Dans ces conditions, après le pic de volume mort vers 3 minutes, la molécule tétrabiotinylée/maléimide sort vers 15 minutes. L'analyse LC/MS (HPLC et détecteur de masse THERMO ELECTRON) permet d'identifier le composé attendu avec un poids moléculaire recalculé à 3617,7 g/mole (théorique 3618.26)

Le reste du brut est purifié en au moins 3 volumes équivalents par injection en HPLC semi-préparative BECKMAN sur une colonne phase inverse C₁₈ VYDAC de 20 mm de diamètre par 250 mm de long, au débit de 22 ml/min. Les éluants A et B sont identiques à ceux utilisés en HPLC analytique. Programme typique de purification : après injection on reste 10 minutes à 5% d'éluant B ; puis passage de 5 à 23 % d'éluant B en 1 minute ; puis gradient de purification de 23 à 31% de B en 30 minutes. Dans ces conditions, les fractions de purification sont prélevées à l'arrivée du massif du produit recherché, à partir d'environ 21 minutes, à raison de 0,33 minute par tube, jusqu'à environ 30 minutes (soit de l'ordre de 25 tubes). Une aliquote de chaque tube est analysée en mode isocratique en HPLC analytique (mêmes conditions que ci-dessus sauf isocratique de 26% d'éluant B pendant 10 minutes).

Les fractions les plus pures sont rassemblées dans un ballon en verre puis cette solution est lyophilisée. Après lyophilisation et pesée, on reprend le lyophilisat dans l'eau déminéralisée à 1 mg/ml en fonction de la pesée. Cette solution mère est répartie dans des flacons en verre brun à raison de 1 à quelques millilitres par flacon. Puis les flacons sont à nouveau lyophilisés et bouchés sous une atmosphère inerte de 700 millibars d'argon.

Une aliquote est utilisée pour procéder aux analyses finales en HPLC (BECKMAN, conditions identiques à la 1^{ère} analyse en gradient ci-dessus), LC/MS (conditions idem ci-dessus) et analyse d'acides aminés selon le mode opératoire du fournisseur sur le système Agilent 1100 *series* avec détection par fluorescence.

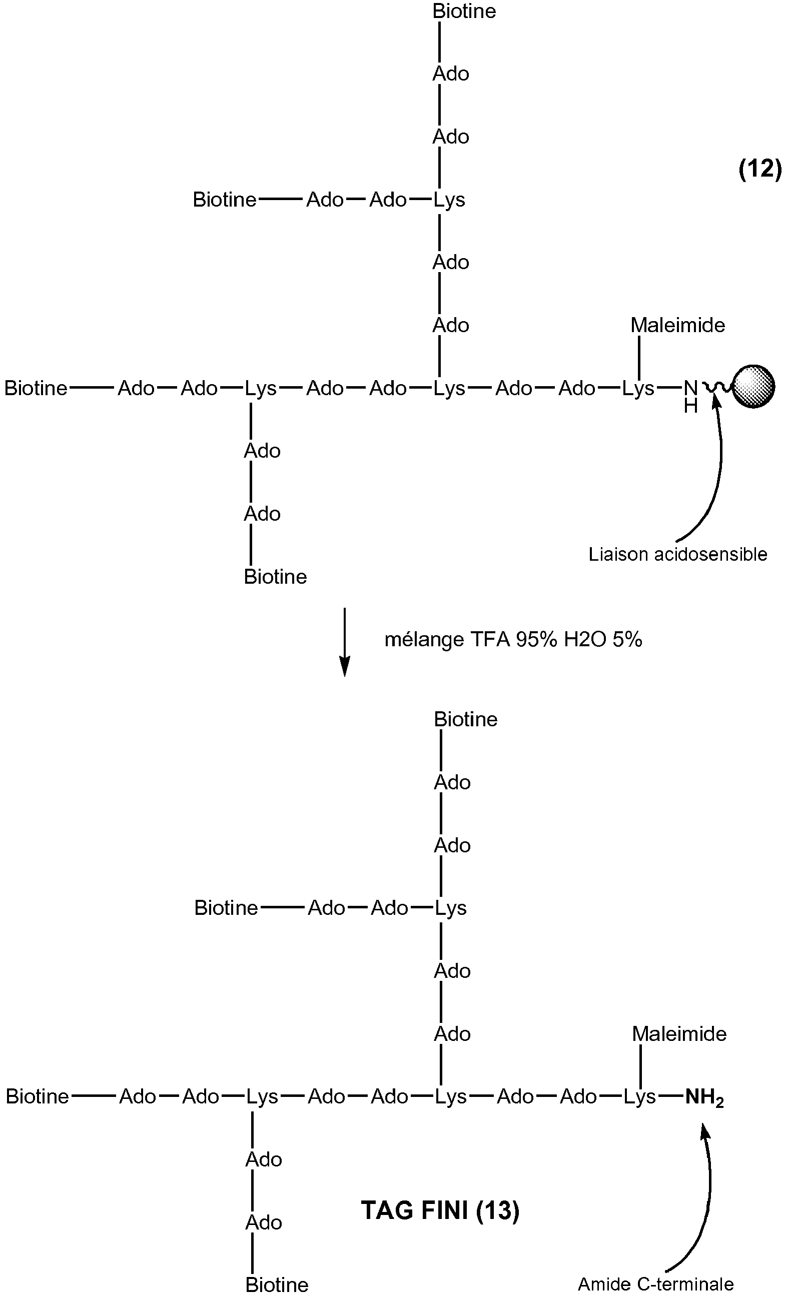

Le composé tétrabiotinylé (13) ainsi obtenu présente la formule développée telle que décrite sur la figure 1.

### Exemple 2 : Préparation d'un composé de formule (I) présentant 4 biotines

(AA=AA₁=AA₂= dérivé de lysine ;
B est NH₂ ;
G₀=G₁=G₂= (-NH-CH₂-CH₂-O-CH₂-CH₂-O-CH₂-CO-) = Ado ;
n₀=n₁=n₂= 3
T= maléimide
X= 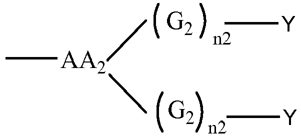
Y = biotine)

Dans cet exemple, on a utilisé les mêmes réactifs que ceux décrits dans l'exemple 1 à l'exception de la résine : on a disposé dans le réacteur du synthétiseur ABI433A environ 148 micromoles de résine H-RINK-ChemMatrix (MATRIX Innovation) qui est sous forme amine libre exempte de groupement Fmoc.

### 2.1 Couplage 1^{ère} lysine (AA) :

A la différence de l'exemple 1, la Fmoc-Lys(Mmt)-OH a été couplée manuellement. La résine est déjà sous forme amine et ne nécessite pas de déprotection. Puis, 137 micromoles de Fmoc-Lys(Mmt)-OH repris en solution dans la NMP ont été envoyés manuellement dans le réacteur avec 1 équivalent de l'agent de couplage HBTU/HOBt. Au final, les réactifs se trouvent dans un volume d'environ 6 ml. Enfin, la réaction de couplage est démarrée par envoi dans le réacteur de 0,5 ml de la même solution de DIEA 2 M / NMP. La résine est en léger excès. On laisse alors réagir 2 heures à température du laboratoire avec vortex intermittent. Après quoi, on reprend la synthèse en mode totalement automatisée selon le programme indiqué dans le tableau 3 suivant :

**Tableau 3**

| **Cycle** | **AA** | **Nom** | **Modules** |
|---|---|---|---|
| 1 | Fmoc-Lys(Mmt)-OH | Fin couplage et masquage des amines résiduelles de la résine | IIDCCDD |
| 2 | Fmoc-Ado-OH | Double couplage conditionnel et capping | BbADEFfiafCd |
| 3 | Fmoc-Ado-OH | Double couplage conditionnel et capping | BbADEFfiafCd |
| 4 | Fmoc-Ado-OH | Double couplage conditionnel et capping | BbADEFfiafCd |
| 5 | Fmoc-Lys(Fmoc)-OH | Double couplage conditionnel et capping | BbADEFfiafCd |
| 6 | Fmoc-Ado-OH | Double couplage conditionnel long et capping | BbADEFfIIiafIICd |
| 7 | Fmoc-Ado-OH | Double couplage conditionnel long et capping | BbADEFfIIiafIICd |
| 8 | Fmoc-Ado-OH | Double couplage conditionnel long et capping | BbADEFfIIiafIICd |
| 9 | Fmoc-Lys(Fmoc)-OH | Double couplage conditionnel long et capping | BbADEFfIIiafIICd |
| 9 | Fmoc-Ado-OH | Double couplage conditionnel très long et capping | BbADEFfIIIIiafIIIICd |
| 10 | Fmoc-Ado-OH | Double couplage conditionnel très long et capping | BbADEFfIIIIiafIIIICd |
| 11 | Fmoc-Ado-OH | Double couplage conditionnel très long et capping | BbADEFfIIIIiafIIIICd |
| 12 | Biotine | Double couplage conditionnel très long et capping | BbADEFfIIIIiafIIIICd |
| 13 | N-maleoyl-βalanine | Déprotection Lys(Mmt) double couplage long et capping | GcADEFIAdEFfICd |
| 14 | Aucun | Lavages résine NMP et DCM | Dcc |

Après le premier cycle de ce programme, on obtient le même type de structure que le composé **(1)** de l'exemple 1.

### 2.2 Construction du squelette

On a répété le mode opératoire décrit dans le paragraphe 1.2 étape i), à ceci près que cette étape i) est répétée 3 fois et par conséquent 3 Fmoc-Ado-OH (et non plus 2) sont couplés successivement pour aboutir à la structure suivante (étape 2.2.i)) :

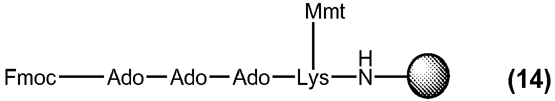

Puis on a procédé à la ramification à l'identique de l'étape 1.2 ii) en couplant Fmoc-Lys(Fmoc)-OH pour obtenir (étape 2.2.ii)) :

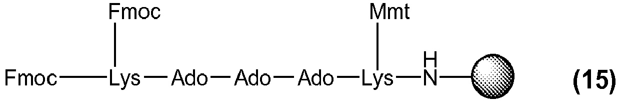

On a ensuite répété à l'identique les étapes 2.2 i) et 2.2 ii) ci-dessus pour aboutir à la molécule suivante à deux ramifications :

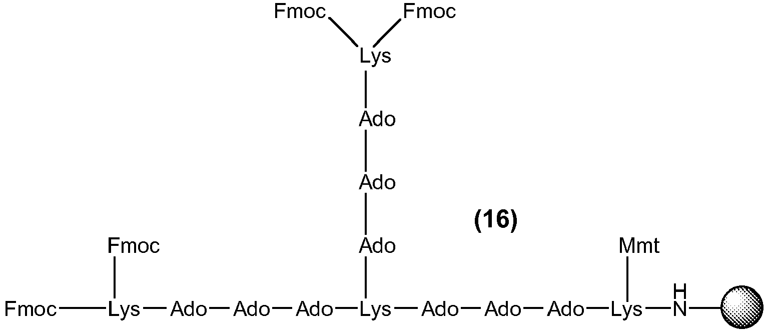

On a de nouveau répété à l'identique les étapes 2.2 i) et 2.2 ii) ci dessus pour aboutir à la molécule intermédiaire à quatre ramifications protégées par des « Fmoc » :

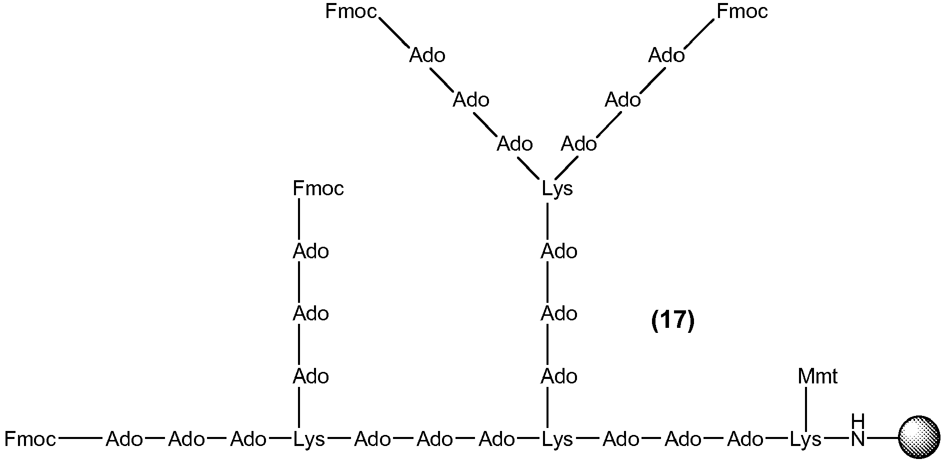

Puis on a procédé comme indiqué aux paragraphes 1.3, 1.4 pour aboutir à la molécule tétrabiotinylée avec maléimide :

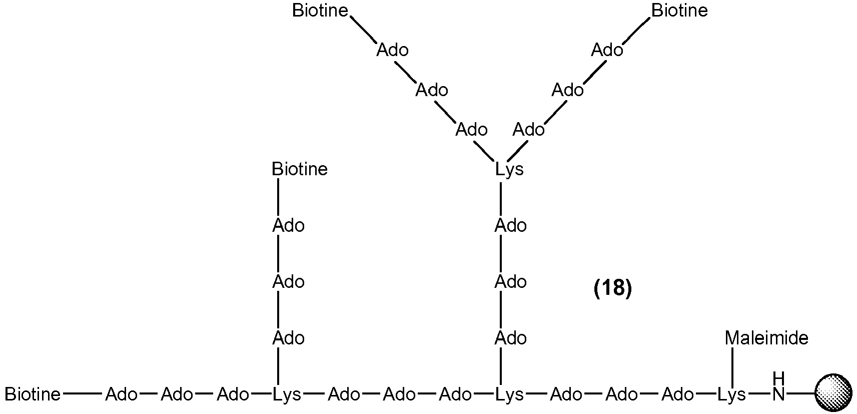

On a effectué le clivage de la molécule de son support, l'analyse et la purification, dans des conditions similaires à celles de l'exemple 1. On a observé la sortie en HPLC analytique (conditions expérimentales identiques) de la molécule tétrabiotinylée / maléimide avec des bras de « 3 Ado » vers 15 minutes. L'analyse LC/MS (conditions identiques) confirme la présence du composé attendu avec un poids moléculaire recalculé à 4634 g/mole (théorique 4634.37). La purification de cette molécule est effectuée dans les conditions identiques à l'exemple 1, à ceci près que le gradient de purification consiste cette fois en un passage de 22 à 32 % de l'éluant B toujours en 30 minutes.

Comme dans l'exemple 1, les fractions les plus pures sont rassemblées et ce mélange est lyophilisé. Après quoi, le lyophilisat est pesé et repris dans l'eau (soluble) et réparti en fractions de 1 à quelques millilitres, à leur tour lyophilisés puis bouchés sous atmosphère inerte de 700 millibars d'argon.

Comme dans l'exemple 1, une aliquote est utilisée pour procéder aux analyses finales identiques.

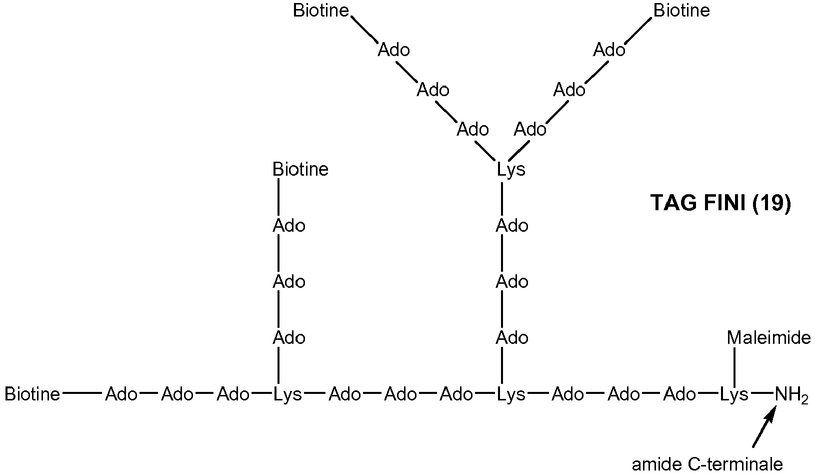

### Exemple 3 : Préparation d'un composé de formule (I) présentant 8 biotines

(AA=AA₁=AA₂= AA₃= dérivé de lysine ;
B est NH₂ ;
G₀=G₁=G₂= G₃=(-NH-CH₂-CH₂-O-CH₂-CH₂-O-CH₂-CO-) = Ado;
n₀=n₁=n₂= 3
T= maléimide
Y = 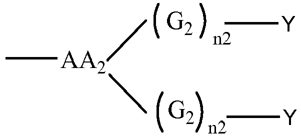
Z = biotine

Pour obtenir cette structure à huit ramifications, on répète la méthode utilisée dans l'exemple 1, à quelques différences près. Le programme utilisé sur le synthétiseur ABI433A est tel qu'indiqué dans le tableau 4 suivant :

**Tableau 4**

| **Cycle** | **AA** | **Nom** | **Modules** |
|---|---|---|---|
| 1 | Fmoc-Lys(Mmt)-OH | Couplage simple en faible stoechiométrie et masquage des amines résiduelles de la résine | BbDgFfIIIIIICCd |
| 2 | Fmoc-Ado-OH | Double couplage conditionnel et capping | BbADEFfiafCd |
| 3 | Fmoc-Ado-OH | Double couplage conditionnel et capping | BbADEFfiafCd |
| 4 | Fmoc-Ado-OH | Double couplage conditionnel et capping | BbADEFfiafCd |
| 5 | Fmoc-Lys(Fmoc)-OH | Double couplage conditionnel et capping | BbADEFfiafCd |
| 6 | Fmoc-Ado-OH | Double couplage conditionnel et capping | BbADEFfiafCd |
| 7 | Fmoc-Ado-OH | Double couplage conditionnel et capping | BbADEFfiafCd |
| 8 | Fmoc-Ado-OH | Double couplage conditionnel et capping | BbADEFfiafCd |
| 9 | Fmoc-Lys(Fmoc)-OH | Double couplage conditionnel et capping | BbADEFfiafCd |
| 9 | Fmoc-Ado-OH | Double couplage conditionnel long et capping | BbADEFfIIiafIICd |
| 10 | Fmoc-Ado-OH | Double couplage conditionnel long et capping | BbADEFfIIiafIICd |
| 11 | Fmoc-Ado-OH | Double couplage conditionnel long et capping | BbADEFfIIiafIICd |
| 12 | Fmoc-Lys(Fmoc)-OH | Double couplage conditionnel long et capping | BbADEFfIIiafIICd |
| 13 | Fmoc-Ado-OH | Double couplage systématique long et capping | BbADEFfIIIAdEFfIIICd |
| 14 | Fmoc-Ado-OH | Double couplage systématique long et capping | BbADEFfIIIAdEFfIIICd |
| 15 | Fmoc-Ado-OH | Double couplage systématique long et capping | BbADEFfIIIAdEFfIIICd |
| 16 | Biotine | Double couplage systématique long et capping | BbADEFfIIIAdEFfIIICd |
| 17 | N-maleoyl-βalanine | Déprotection Lys(Mmt) double couplage long et capping | GcADEFIAdEFfICd |
| 18 | Aucun | Lavages résine NMP et DCM | Dcc |

Le chargement de 448 micromoles de résine (Fmoc-RINK-MBHA) est effectué automatiquement mais par seulement 80 micromoles de Fmoc-Lys(Mmt)-OH pour aboutir au composé sur résine **(1)** de l'exemple 1. L'excès d'amine sur la résine est masqué par acétylation.

Puis la synthèse continue à l'identique de l'exemple 2, jusqu'à l'étape permettant d'obtenir la structure **(17).**

A partir de là, on effectue la répétition à l'identique des étapes 2.2 i) et 2.2 ii) de l'exemple 2, pour aboutir à la molécule intermédiaire à huit ramifications protégées par des groupements « Fmoc » :

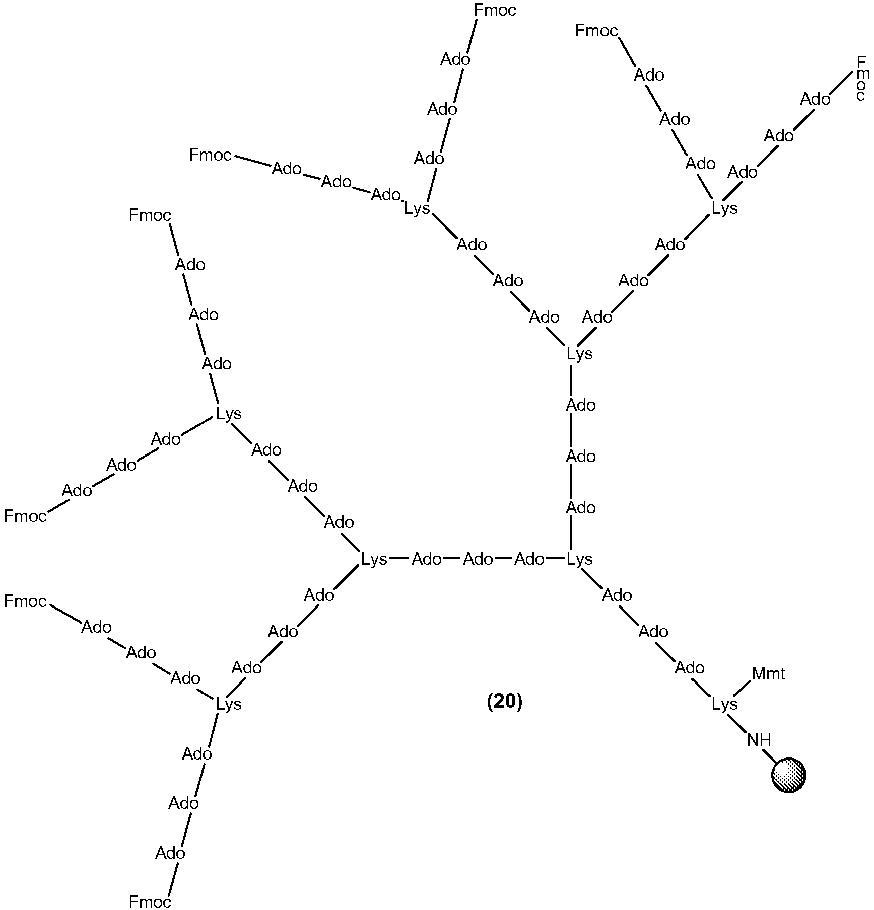

Puis on procède comme pour l'exemple 1, aux étapes identiques aux paragraphes 1.3, 1.4 de l'exemple 1, pour aboutir à la molécule octobiotinylée avec maléimide :

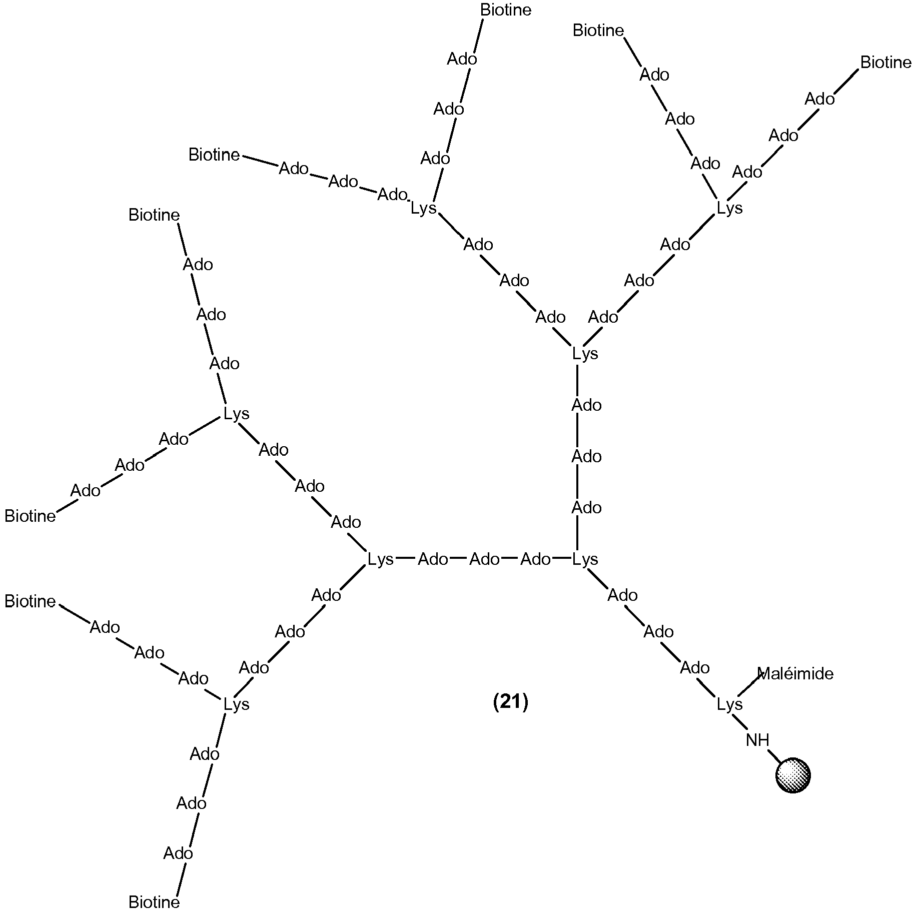

Le clivage de la molécule de son support est effectué dans des conditions similaires à l'exemple 1.5, ainsi que l'analyse et la purification. En HPLC analytique (conditions expérimentales identiques), la molécule octobiotinylée/maléimide avec des bras de 3 Ado sort sous forme de pic large vers 16 minutes. L'analyse LC/MS (conditions identiques) confirme la présence du composé attendu avec un poids moléculaire recalculé à 9535 g/mole (théorique 9536,06). La purification de cette molécule est effectuée dans les conditions identiques à l'exemple 1, à ceci près que le gradient de purification consiste en un passage de 25 à 35 % de l'éluant B en 30 minutes.

Comme dans l'exemple 1, les fractions les plus pures sont rassemblées et ce mélange est lyophilisé. Après quoi, le lyophilisat est pesé et repris dans l'eau (soluble) et réparti en fractions de 1 à quelques millilitres, à leur tour lyophilisés puis bouchés sous atmosphère inerte de 700 millibars d'argon.

Comme dans l'exemple 1, une aliquote est utilisée pour procéder aux analyses finales identiques.

La molécule octobiotinylée/maléimide avec bras de « 3 Ado » obtenue est la molécule **(22)** suivante :

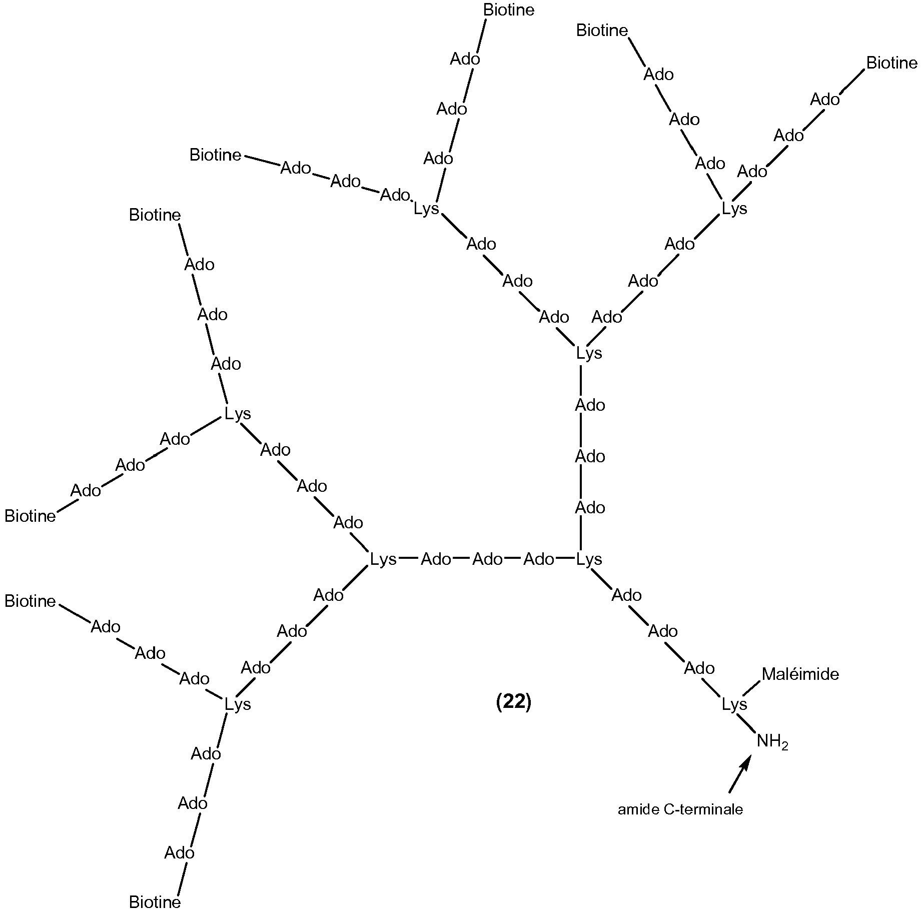

### Exemple 4 : Couplage de protéines-SH avec un composé de formule (I)

### 4.1. Contexte

Le couplage à la biotine sur des protéines-SH (par exemple fragments Fab', antigènes réduits au DTT et protéines modifiées par le réactif de Traut) est réalisé traditionnellement en utilisant de la biotine BMCC (Pierce ref 21900 : 1-Biotinamido-4{4'-(maléimidomethyl)cyclohexanecarboxamido]butane) qui se fixe sur les fonctions sulfhydryle libres des protéines SH. Toutefois, la faible quantité de fonctions sulfhydryle libres ne permet pas de fixer un nombre important de biotine, de sorte que quand le conjugué biotine/protéine-SH est utilisé en diagnostic, le signal de détection est faible, représentatif que de la présence d'une biotine par conjugué. Il convient donc d'augmenter le signal.

Les composés de l'invention permettent de lier de 2 à 32 biotines et la Demanderesse a montré que, contre toute attente, ils conservaient un signal proportionnel au nombre de biotines présents, après couplage aux radicaux sulfhydryle libres des protéines-SH, et ce malgré leur encombrement stérique du fait du grand nombre de biotines liées.

Le protocole de couplage est détaillé dans les paragraphes suivants.

### 4.2. Réactifs

Les réactifs du commerce utilisés sont indiqués dans le tableau 5.

**Tableau 5**

| **Noms** | **Fournisseurs** | **Références** |
|---|---|---|
| Pepsine agarose | Sigma | P0609-50KU |
| 2 β mercapto ethanol | Thermo scientific | 35602 |
| Biotine BMCC | Thermo scientific | 21900 |
| DMSO | Merck | 1.02950.0500 |
| Iodoacétamide | Sigma | I6125 |
| Réactif de TRAUT | Thermo scientific | 26101 |
| N-Ethylmaleimide | Sigma | E-3876 |
| DTT | Sigma | D0632 |
| Biotine NHs | Roche | 11003933 |

Par ailleurs, les protéines-SH utilisées, obtenues de bioMérieux, France, sont les suivantes :
- Fragments Fab' : fragment Fab' anti-*Listeria monocytogenes,* anti-antigène de paroi de Salmonelle, anti-p24 de HIV clone A et clone B et anti-Kallikréine humaine 2 ;
- Antigènes utilisés : gp160 de HIV, HBs Ad du HBV, HBs Ay du HBV
- Anticorps : anticorps anti-antigène de paroi de Salmonelle.

Les antigènes utilisés pour le dosage ELISA indirect des fragments Fab' de Salmonelle ont été obtenus après culture des souches de *Salmonella bergedorf, typhimurium et enteritidis* en milieu peptonée pendant 24h à 37°C, puis chauffage pendant 15 min à 100°C.

Enfin, on a utilisé le composé de formule (I) préparé selon l'exemple 1.

### 4.3. Couplage à la biotine BMCC et au composé de l'exemple 1 sur des Fab'

On a obtenu les conjugués par couplage comme suit :
- obtention du fragment Fab' par digestion pepsique, puis réduction au 2βME, ajusté à 2 mg/ml après purification sur Superdex 200 prep grade, en tampon PO₄ 50mM + NaCl 150 mM + EDTA(4Na) 5mM pH=6.8,
- préparation d'une solution extemporanée de biotine BMCC (533,69 g/mole) à 8.5 mM (4.54 g/l) en DMSO,
- préparation d'une solution extemporanée de composé de l'exemple 1 (3618,26 g/mole) à 2.5 mg/ml en eau déminéralisée,
- couplage au Fab' à raison de 5 moles biotine BMCC ou composé de l'exemple 1 par mole de Fab' (46 000 Daltons),
- incubation 2 heures à température du laboratoire avec agitation rotative en flacon verre brun,
- blocage par addition de iodoacétamide équimolaire à la biotine BMCC ou au composé de l'exemple 1,
- utilisation d'une solution extemporanée de iodoacétamide 10 mM en tampon PBS,
- incubation 1H à température du laboratoire avec agitation rotative,
- dialyse contre tampon PBS + azide,
- sortie de dialyse et détermination de la concentration des conjugués par mesure de la DO à 280nm (ε = 1,48)

### 4.4. Couplage du composé de l'exemple 1 sur des anticorps modifiés par le Traut

On a obtenu les conjugués par couplage comme suit :
- modification de l'anticorps par addition de 20 moles de Traut pour 1 mole d'anticorps, puis dialyse en tampon PO₄ 50mM + NaCl 150 mM + EDTA(4Na) 5mM pH=6.8,
- préparation d'une solution extemporanée de composé de l'exemple 1 (3618,26 g/mole) à 2.5 mg/ml en eau déminéralisée,
- couplage à l'anticorps modifié à raison de 20 moles composé de l'exemple 1 par mole d'anticorps (160 000 Daltons),
- incubation 2 heures à température du laboratoire avec agitation rotative en flacon verre brun,
- blocage par addition de iodoacétamide équimolaire au composé de l'exemple 1,
- utilisation d'une solution extemporanée de iodoacétamide en PBS,
- incubation 1H à température du laboratoire avec agitation rotative,
- dialyse contre tampon PBS + azide,
- sortie de dialyse et détermination de la concentration du conjugué par mesure de la DO à 280nm (ε = 1,4)

### 4.5. Couplage du composé de l'exemple 1 sur des antigènes modifiés par le Traut

On a obtenu les conjugués par couplage comme suit :
- modification de l'antigène par addition de 4 moles de Traut pour 1 mole d'antigène, puis dialyse en tampon PO₄ 50mM + NaCl 150 mM + EDTA(4Na) 5mM pH=6,8,
- préparation d'une solution extemporanée de composé de l'exemple (3618,26 g/mole) à 1 mg/ml en eau déminéralisée,
- couplage à l'antigène modifié à raison de 1 moles de composé de l'exemple 1 par mole d'antigène (PM = 25 000 Daltons),
- incubation 2 heures à température du laboratoire avec agitation rotative en flacon verre brun,
- blocage par addition de iodoacétamide équimolaire au composé de l'exemple 1,
- utilisation d'une solution extemporanée de iodoacétamide en PBS,
- incubation 1H à température du laboratoire avec agitation rotative,
- dialyse contre tampon PBS + azide + SDS (sodium dodécylsulfate),
- sortie de dialyse et détermination de la concentration des conjugués par mesure de la DO à 280nm.

### 4.6. Couplage de la biotine NHs sur des antigènes ou des anticorps

On a obtenu les conjugués par couplage comme suit :
- dialyse des anticorps ou des antigènes en tampon NaHCO₃ 0,1M pH=8,3
- préparation d'une solution extemporanée de biotine NHs à 11,36 g/l en DMSO pour le couplage des anticorps et d'une solution à 2,0 g/l en DMSO pour le couplage des antigènes
- couplage à l'anticorps à raison de 20 moles biotine NHs par mole d'anticorps (160 000 Daltons), et couplage à l'antigène à raison de 1 mole biotine NHs par mole antigène (25 000 daltons)
- incubation 1 heure à température ambiante avec agitation rotative en flacon verre brun,
- blocage par addition de lysine 1M pH=8,0 équimolaire à la biotine NHs,
- incubation 1h à température ambiante avec agitation rotative,
- dialyse contre tampon PBS + azide pour les anticorps et en PBS + azide + SDS pour les antigènes,
- sortie de dialyse et détermination de la concentration du conjugué par mesure de la DO à 280nm.

### 4.7. Couplage du composé de l'exemple 1 et de la biotine BMCC sur des antigènes réduits au DTT

On a obtenu les conjugués par couplage comme suit :
- réduction de l'antigène par addition de 800 moles de DTT par mole d'antigène et agitation pendant 30 min à température du laboratoire,
- dessalage de l'antigène réduit sur gel G25 Sephadex en tampon PBS + EDTA pH=7,5,
- préparation d'une solution extemporanée de biotine BMCC (533,69 g/mole) à 4,35 mM (2,32 g/l) en DMSO,
- préparation d'une solution extemporanée du composé de l'exemple 1 (3618.26 g/mole) à 2.5 mg/ml en eau déminéralisée,
- couplage à l'antigène réduit à raison de 20 moles de biotine BMCC ou de composé de l'exemple 1 par mole d'antigène (160 000 Daltons),
- incubation 2 heures à température du laboratoire avec agitation sur vortex en flacon verre brun,
- blocage par addition de 20 moles de NEM par mole d'antigène,
- utilisation d'une solution extemporanée 10mM de NEM en tampon PBS,
- dialyse des conjugués en PBS + MIT,
- sortie de dialyse.

### Exemple 5 : Application en tests de détection directe et indirecte ELISA

### 5.1. Contrôle ELISA direct

L'activité des conjugués présentant un composé de l'invention tétrabiotinylée ou une seule biotine est comparée avec un test ELISA direct, en fixant les conjugués sur microplaque et en révélant le signal par l'intermédiaire de la streptavidine couplée à la peroxydase. Cet essai permet de comparer le "niveau de couplage" des composés polybiotinylés, selon un mode de réalisation de l'invention, avec des composés monobiotinylés.

### Protocole :

Utilisation des barrettes Maxisorp (pour dépôt de fragments d'anticorps et anticorps entiers) ou Polysorp (pour dépôt des antigènes).

Dilution des conjugués monobiotinylés ou tétrabiotinylés entre 0,05 µg/ml et 0.5 µg/ml en tampon CO₃ 50 mM pH=9.6, puis dilutions de 2 en 2.
Dépôt 100 µl/puits de chaque dilution
Incubation 1 nuit à température du laboratoire
3 lavages en PBS tween
Dépôt 100 µl/puits de streptavidine-peroxydase diluée au 1/6000° en PBS
Incubation 15 min à l'étuve à 37°C
3 lavages en PBS tween
Dépôt 100 µl/puits OPD
Incubation 30 min à l'obscurité à température du laboratoire
Blocage avec 100 µl/puits H₂SO₄ 1.8N
Lecture de la DO à 492 nm

### 5.2. Contrôle ELISA indirect avec un conjugué Fab' anti-Salmonelle/composé tétrabiotinylé ou monobiotinylé

Pour ce faire, les antigènes issus de la culture des 3 souches de Salmonelle ont été coatés sur des microplaques Polysorp. Puis on a réalisé les étapes suivantes :
- passivation 1H à l'étuve à 37°C avec de la BSA 1 g/l en PBS,
- 3 lavages PBStween, puis
- incubation des conjugués dilués de 2 en 2 en PBS pendant 30 min à 37°C, 3 lavages PBS tween.

On a ensuite procédé comme pour l'ELISA direct à partir du dépôt de streptavidine.

L'ELISA indirect permet à la fois de tester le "niveau de couplage" et la réaction biologique entre le conjugué et l'antigène ou l'anticorps.

### 5.3 Résultats

Les Résultats sont reportés sur les Figures 2 à 8 comme suit :
- la figure 2 est la représentation graphique donnant les résultats de DO du test ELISA direct du conjugué Fab' anti-*Listeria monocytogenes* (LMO) monobiotinylé (par de la biotine BMCC) ou tétrabiotinylé (par un composé selon un mode de réalisation de l'invention), fixé au fond de la microplaque, en fonction de sa concentration,
   la figure 3 est la représentation graphique donnant les résultats de DO du test ELISA direct du conjugué Fab' anti-Salmonelle monobiotinylé (par de la biotine BMCC) ou tétrabiotinylé (par un composé selon un mode de réalisation de l'invention), fixés au fond de la microplaque, en fonction de leur concentration, ainsi que du test ELISA indirect de ces mêmes conjugués, mais détectés par l'intermédiaire d'antigènes de Salmonelles,
- la figure 4 est la représentation graphique donnant les résultats de DO du test ELISA direct de deux conjugués Fab' anti-HIV monobiotinylés (par de la biotine BMCC) ou tétrabiotinylés (par un composé selon un mode de réalisation de l'invention), fixés au fond de la microplaque, en fonction de leur concentration,
- la figure 5 est la représentation graphique donnant les résultats de DO du test ELISA direct du conjugué Fab' anti-Kallikréine monobiotinylé (par de la biotine BMCC) ou tétrabiotinylé (par un composé selon un mode de réalisation de l'invention), fixé au fond de la microplaque, en fonction de sa concentration,
- la figure 6 est la représentation graphique donnant les résultats de DO du test ELISA direct du conjugué antigène gp160 réduit au DTT monobiotinylé (par de la biotine BMCC) ou tétrabiotinylé (par un composé selon un mode de réalisation de l'invention), fixé au fond d'une microplaque, en fonction de sa concentration,
- la figure 7 est la représentation graphique donnant les résultats de DO du test ELISA direct des conjugués antigènes de surface de l'hépatite B monobiotinylés (par de la biotine NHs) ou tétrabiotinylés (par un composé selon un mode de réalisation de l'invention), fixés au fond de la microplaque, en fonction de leur concentration, et
- la figure 8 est la représentation graphique donnant les résultats de DO du test ELISA direct du conjugué anticorps anti-Salmonelle monobiotinylé (par de la biotine NHs) ou tétrabiotinylé (par un composé selon un mode de réalisation de l'invention), fixé au fond de la microplaque, en fonction de sa concentration.

Les résultats mettent en évidence une nette amélioration du signal en utilisant la molécule polybiotinylée selon l'invention, malgré son encombrement. En particulier, pour une DO de 1,8, on peut observer les gains tels qu'indiqués dans le tableau 6 suivant :

**Tableau 6**

| | Biotine BMCC | Biotine NHs | Tétrabiotine | Gain signal |
|---|---|---|---|---|
| LMO Essai 1 | 46 ng/ml | NA | 6 ng/ml | × 8 |
| LMO Essai 2 | 6.0 ng/ml | NA | 0.4 ng/ml | × 15 |
| HIV Anticorps A | 0.078 µg/ml | NA | <0.0078 µg/ml DO = 1.895 | > × 10 |
| HIV Anticorps B | 0.08 µg/ml | NA | <0.0078 µg/ml DO = 2.021 | > × 10 |
| Kallikréine | 0.287 µg/ml | NA | <0.0023 µg/ml DO = 2.226 | > × 124 |
| Salmonelle Fab' ELISA direct | 0.012 µg/ml | NA | <0.0039 µg/ml DO = 2.825 | > × 30 |
| Salmonelle Fab' ELISA indirect | 0.226 µg/ml | NA | 0.101 µg/ml | × 2.2 |
| Antigène GP160 réduit au DTT | 0.01 µg/ml | NA | <0.0039 µg/ml DO = 2.813 | > × 2.5 |
| Antigène HBs Ad | NA | 5 µg/ml DO = 1.320 | Traut (1/4) Tétrabiotine (1/1) 0.063 µg/ml | < × 79 |
| Antigène HBs Ay | NA | 2.27 µg/ml | Traut (1/4) Tétrabiotine (1/1)0.046 µg/ml | × 49 |
| Anticorps de Salmonelle | NA | (1/20) 0.19 µg/ml | Traut (1/20) Tétrabiotine (1/20) 0.029 µg/ml | × 6.5 |

### Exemple 6 : Application en tests diagnostiques à partir de souches de Listeria

L'objectif de cet essai est de comparer le signal Vidas obtenu entre un conjugué tétrabiotinylé selon un mode de réalisation et un conjugué monobiotinylé, dans le but d'augmenter la sensibilité de la réponse sans dégrader la spécificité.

### 6.1 Matériels et Méthodes

### Réactifs utilisés

Les conjugués tétrabiotinylés et monobiotinylés ont été préparés en utilisant un fragment d'anticorps Fab' de *Listeria monocytogenes* (bioMérieux, France) et respectivement de la biotine BMCC et un composé tel que préparé dans l'exemple 1, selon le protocole décrit au point 4.3 ci-dessus.

Les autres réactifs sont les suivants :
- Cônes et barrettes LMO2 (bioMérieux, Référence 30 704)
- Streptavidine PAL (BioSPA, Réf : 045 66074)
- Solution mère monobiotinylé : concentration 0,32 mg/ml de Fab'
- Solution mère tétrabiotinylé : concentration 0,26 mg/ml de Fab'
- Diluant conjugué LMO2 (bioMérieux, Réf 500 26004)
- Diluant Streptavidine PAL (bioMérieux, Réf 500 25992)
- 1 appareil Vidas (bioMérieux)

### Souches testées

- Listeria monocytogenes 4b ATCC 19115
- Listeria monocytogenes 3a ATCC 51 782
- Listeria monocytogenes 1/2c 83 09 024
- Listeria monocytogenes 4c 83 09 031
- Listeria innocua 6a 83 09 035
- Listeria ivanovii 91 01 014
- Listeria welshimerii 94 09 074

### Méthodes

Toutes les souches ont été cultivées 24h à 37°C ± 1°C en bouillon LX Réf 42 120, puis chauffées 5' à 95-100°C.

Les souches de *Listeria innocua, Listeria ivanovii* et *Listeria welshimerii* sont utilisées pures. Ces souches permettent de tester la spécificité puisqu'elles ne sont pas reconnues par l'anticorps couplé.

Les quatre souches de *Listeria monocytogenes* sont testées en dilution et ont fait l'objet d'un dénombrement. Ces souches permettent de tester la sensibilité du test.

Les 2 conjugués ont été ajustés à la concentration de 0,36µg/ml de Fab'.

Pour utiliser le kit de la barrette LMO2, le conjugué initial a été retiré du puits X5. Ce puits a été rincé avec 600µl d'eau physiologique. Puis, après avoir retiré l'eau physiologique, 400µl soit du conjugué monobiotinylé, soit du conjugué tétrabiotinylé à tester ont été ajoutés. 500µl d'échantillon (dilution de souches) ont été déposés dans le puits X0 et le test Vidas LMO2 a été lancé. Le test dure environ 80 minutes.

### Résultats

Les résultats sont donnés dans le tableau 7 ci-dessous.

**Tableau 7**

| Souches et dénombrement | | | | | Solutions mères | |
|---|---|---|---|---|---|---|
| | | | | | Tétrabiotinylée | Monobiotinylée |
| | | | | | RFV* | |
| L.mono | 4b | ATCC | 19 | 115 | 9957 | 9817 |
| 1.5.10⁵ | | | | | | |
| L.mono | 4b | ATCC | 19 | 115 | 4350 | 2285 |
| 1.5.10⁴ | | | | | | |
| L.mono | 4b | ATCC | 19 | 115 | 199 | 233 |
| 1.5.10³ | | | | | | |
| L.mono | 3a | ATCC | 51 | 782 | 8517 | 6958 |
| 1.6.10⁵ | | | | | | |
| L.mono | 3a | ATCC | 51 | 782 | 1612 | 829 |
| 1.6.10⁴ | | | | | | |
| L.mono | 3a | ATCC | 51 | 782 | 160 | 78 |
| 1.6.10³ | | | | | | |
| L.mono | 1/2c | 83 | 09 | 024 | 9806 | 8779 |
| 2.1.10⁵ | | | | | | |
| L.mono | 1/2c | 83 | 09 | 024 | 2880 | 1472 |
| 2.1.10⁴ | | | | | | |
| L.mono | 1/2c | 83 | 09 | 024 | 309 | 151 |
| 2.1.10³ | | | | | | |
| L.mono | 4c | 83 | 09 | 031 | 2413 | 1427 |
| 8.4.10⁴ | | | | | | |
| L.mono | 4c | 83 | 09 | 031 | 143 | 82 |
| 8.4.10³ | | | | | | |
| L.mono | 4c | 83 | 09 | 031 | 19 | 10 |
| 8.4.10² | | | | | | |
| L innocua 6a 83 09 035 | | | | | 7 | 9 |
| L ivanovii 91 01 014 | | | | | 7 | 3 |
| L welshimerii 94 09 074 | | | | | 10 | 6 |

| | | | | | | |
|---|---|---|---|---|---|---|
| * Relative Fluorescent Value | | | | | | |

Les résultats dans le tableau ci-dessus montrent que l'utilisation d'un conjugué présentant un composé tétrabiotinylé selon un mode de réalisation de l'invention permet de doubler le signal, et donc d'augmenter la sensibilité du test. Parallèlement, son utilisation n'altère pas la spécificité puisque, dans ce cas, les signaux sont identiques.

## Revendications

1. Procédé de préparation d'un composé de formule (I) : 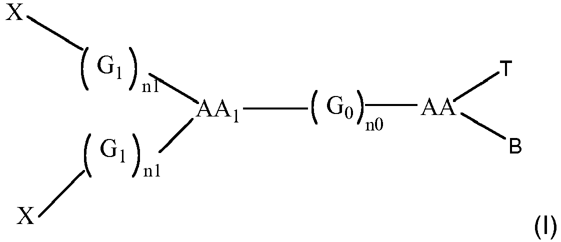 dans laquelle
- X est la biotine ou 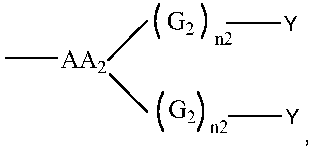
- Y est la biotine ou 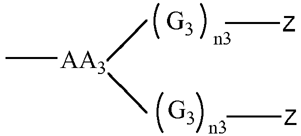
- Z est la biotine ou 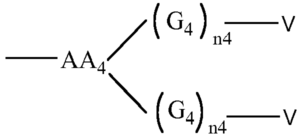
- V est la biotine ou 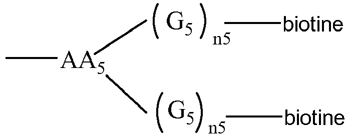
- B est NH₂ ou H,
- AA est un dérivé de molécule trifonctionnelle quand B est NH₂ ou un dérivé de molécule difonctionnelle quand B est H,
- AA₁ à AA₅ sont chacun indépendamment un dérivé de molécule trifonctionnelle,
- G₀ à G₅ sont chacun indépendamment un bras comprenant au moins une unité (-CH₂-CH₂-O),
- n₀ à n₅ sont chacun indépendamment un nombre entier compris entre 1 et 8 et
- T est un antiligand choisi parmi : haptène, anticorps, antigène, peptide, sucre, lectine et polynucléotide ou un groupement réactif pour la fixation à un antiligand, ledit antiligand étant susceptible de réagir avec un ligand,
**caractérisé en ce qu'**il comprend les étapes consistant à :
(i) greffer n₀ composés de formule W"₀-G₀-OH, dans laquelle W"₀ est un groupement protecteur des amines, n₀ et G₀ étant tels que définis précédemment, à un composé de formule (II) : 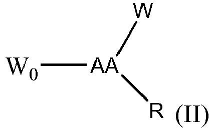 dans laquelle R est une résine préfonctionnalisée, W et W₀ sont différents l'un de l'autre et représentent un groupement protecteur des amines, W étant différent de W"₀, et AA est tel que défini précédemment,
pour obtenir un composé de formule (III) : 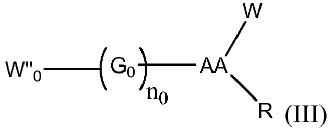
(ii) coupler un composé de formule (IV) : 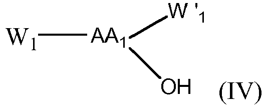 dans laquelle W₁ et W'₁ sont des groupements protecteurs des amines identiques ou différents l'un de l'autre et des groupements W₀ et W"₀, mais différents de W, et AA₁ est tel que défini précédemment, avec le composé de formule (III) pour obtenir un composé de formule (V) : 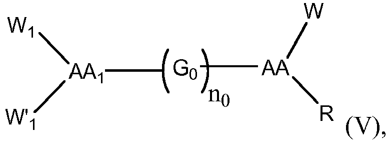
(iii) greffer 2n₁ composés de formule W"₁-G₁-OH, dans laquelle W"₁ est un groupement protecteur des amines, identique ou différent l'un de l'autre et des autres groupements protecteurs utilisés dans ce procédé, mais différents de W, G₁ et n₁ étant tels que définis précédemment, au composé obtenu dans l'étape (ii) pour obtenir un composé de formule (VI) : 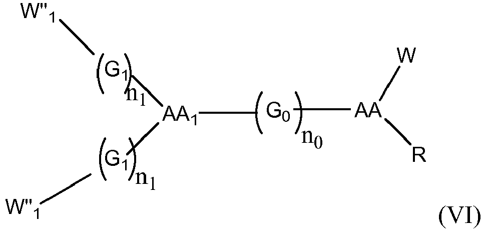
(iv) quand X de la formule (I) est la biotine, passer directement à l'étape (v) ; sinon, quand X de la formule (I) n'est pas la biotine, répéter les étapes (ii) et (iii) avec 2^{p-1} composés de formule (VII) : 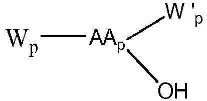 et 2^{p}nₚ fois le composé de formule W"ₚ-Gₚ-OH, dans laquelle p est un nombre entier compris entre 2 et 5, et Wₚ, W'ₚ, W"ₚ sont des groupements protecteurs des amines identiques ou différents les uns des autres et des autres groupements protecteurs utilisés dans ce procédé, mais différents de W, et AA_{P} est une molécule trifonctionnelle, selon la séquence suivante :
- 1 fois quand Y est la biotine, p étant alors égal à 2
- 2 fois quand Y n'est pas la biotine et Z est la biotine, p étant alors égal à 2 puis à 3,
- 3 fois quand Y et Z ne sont pas la biotine et V est la biotine, p étant alors égal à 2, 3 puis 4, et
- 4 fois quand Y, Z et V ne sont pas la biotine, p étant alors égal à 2, 3, 4 puis 5,
(v) déprotéger le composé ainsi obtenu au niveau du groupement W"₁ ou W"ₚ, p étant compris entre 2 et 5, et coupler avec de la biotine,
(vi) déprotéger, au niveau du groupement W, le composé ainsi polybiotinylé et coupler avec un antiligand ou un groupement réactif pour la fixation à un antiligand (T) et
(vii) couper le composé ainsi obtenu de la résine (R) pour obtenir un composé de formule (I).

2. Procédé selon la revendication 1, **caractérisé en ce que** les substituants AA₁ et AA₂, le cas échéant AA₃, AA₄ et AA₅, sont identiques et sont de préférence un dérivé de lysine.

3. Procédé selon l'une quelconque des revendications 1 ou 2, **caractérisé en ce que** les substituants G₀, G₁, le cas échéant G₂, G₃, G₄ et G₅ sont identiques et sont de préférence de formule (-NH-CH₂-CH₂-O-CH₂-CH₂-O-CH₂-CO-).

4. Procédé selon l'une des revendications précédentes, **caractérisé en ce que** les nombres entiers n₀, n₁, le cas échéant n₂, n₃, n₄ et n₅, sont identiques et sont de préférence égaux à 2 ou 3.

5. Procédé selon l'une quelconque des revendications précédentes, **caractérisé en ce que** le groupement W est le méthoxytrityle.

6. Procédé selon l'une quelconque des revendications précédentes, **caractérisé en ce que** W₁ et W'₁ sont identiques et, le cas échéant, W₂ et W'₂ sont identiques, W₃ et W'₃ sont identiques, W₄ et W'₄ sont identiques et W₅ et W'₅ sont identiques.

7. Procédé selon la revendication 6, **caractérisé en ce que** les groupements protecteurs d'amine W₁, W'₁ et, le cas échéant, W₂, W'₂, W₃, W'₃, W₄ et W'₄, W₅ et W'₅ sont des groupements fluorénylméthoxycarbonyle.

8. Procédé l'une quelconque des revendications précédentes, **caractérisé en ce que** le substituant AA est un dérivé de lysine et B est NH₂.

9. Procédé selon l'une quelconque des revendications précédentes, **caractérisé en ce que** X est 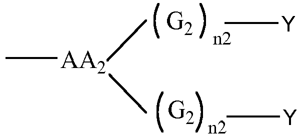 et Y est la biotine.

10. Composé de formule (I) : 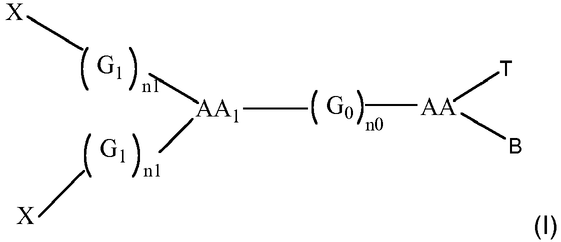 dans laquelle
- X est la biotine ou 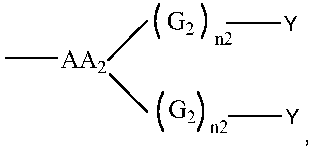
- Y est la biotine ou 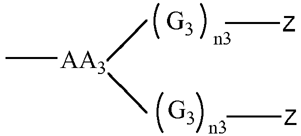
- Z est la biotine ou 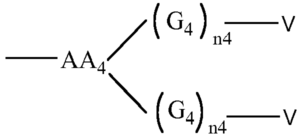
- V est la biotine ou 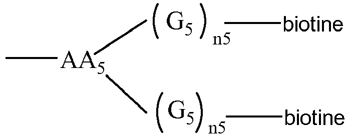
- B est NH₂ ou H,
- AA est un dérivé de molécule trifonctionnelle quand B est NH₂ ou un dérivé de molécule difonctionnelle quand B est H,
- AA₁ à AA₄ sont chacun indépendamment un dérivé de molécule trifonctionnelle,
- G₀ à G₅ sont chacun indépendamment un bras comprenant une unité (-CH₂-CH₂-O-),
- n₀ à n₅ sont chacun indépendamment un nombre entier compris entre 1 et 8 et
- T est un groupement maléimide, un groupement acide carboxylique ou un antiligand choisi parmi : haptène, anticorps, antigène, peptide, sucre, lectine et polynucléotide ; ladite molécule trifonctionnelle comportant trois fonctions identiques ou différentes choisies parmi NH₂ et COOH.

11. Composé de formule (I) selon la revendication 10, **caractérisé en ce que** les substituants AA₁ et AA₂, le cas échéant AA₃, AA₄ et AA₅, sont identiques et sont de préférence un dérivé de lysine.

12. Composé de formule (I) selon l'une quelconque des revendications 10 et 11, **caractérisé en ce que** les substituants G₀, G₁, le cas échéant G₂, G₃, G₄ et G₅, sont identiques et sont de préférence de formule (-NH-CH₂-CH₂-O-CH₂-CH₂-O-CH₂-CO-).

13. Composé de formule (I) selon l'une des revendications 10 à 12, **caractérisé en ce que** les nombres entiers n₀, n₁, le cas échéant n₂, n₃, n₄ et n₅, sont identiques et sont de préférence égaux à 2 ou 3.

14. Composé de formule (I) selon l'une quelconque des revendications 10 à 13, **caractérisé en ce que** X est 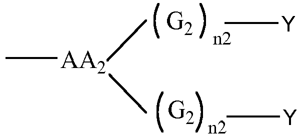 et Y est la biotine.

15. Composé de formule (I) selon l'une quelconque des revendications 10 à 14, **caractérisé en ce que** AA est un dérivé de lysine et B est NH₂.

16. Composé de formule (I) selon l'une quelconque des revendications 10 à 15, **caractérisé en ce que** l'antiligand est un fragment Fab'.

17. Composé de formule (I) selon la revendication 10, **caractérisé en ce que** :
- X est 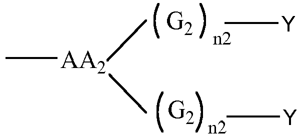
- Y est la biotine,
- AA, AA₁ et AA₂ sont un dérivé de lysine,
- G₀, G₁ et G₂ sont de formule -(NH-CH₂-CH₂-O-CH₂-CH₂-O-CH₂-CO)-,
- n₀, n₁ et n₂ sont égaux à 2,
- B est NH₂ et
- T est un antiligand, de préférence un fragment Fab'.

18. Utilisation des composés obtenus par le procédé tel que décrit dans les revendications 1 à 9 pour l'amplification de signal dans un test diagnostique.

19. Utilisation des composés tels que décrits dans les revendications 10 à 17 pour l'amplification de signal dans un test diagnostique.

## Patentansprüche

1. Ein Verfahren zur Herstellung einer Verbindung der Formel (I): 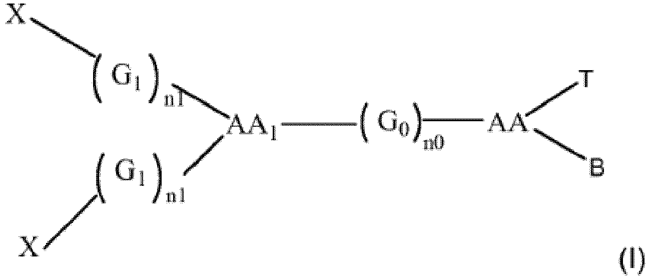 wobei
- X Biotin oder Folgendes ist: 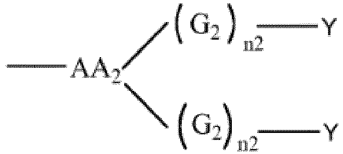
- Y Biotin oder Folgendes ist: 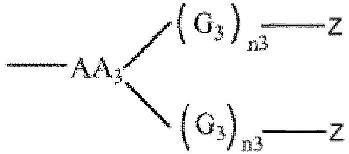
- Z Biotin oder Folgendes ist: 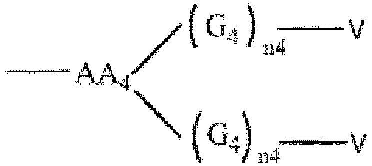
- V Biotin oder Folgendes ist: 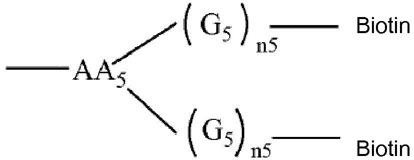
- B NH₂ oder H ist,
- AA ein Derivat eines trifunktionellen Moleküls ist, wenn B NH₂ ist, oder ein Derivat eines difunktionellen Moleküls ist, wenn B H ist,
- AA₁ bis AA₅ jeweils unabhängig ein Derivat eines trifunktionellen Moleküls sind,
- G₀ bis G₅ jeweils unabhängig ein Arm, beinhaltend mindestens eine (-CH₂-CH₂-O)-Einheit, sind,
- n₀ bis n₅ jeweils unabhängig eine ganze Zahl von 1 bis 8 sind und
- T ein Antiligand, der aus Hapten, Antikörper, Antigen, Peptid, Zucker, Lektin und Polynukleotid ausgewählt ist, oder eine reaktive Gruppe zur Bindung an einen Antiliganden ist, wobei der Antiligand in der Lage ist, mit einem Liganden zu reagieren,
**dadurch gekennzeichnet, dass** es die folgenden Schritte beinhaltet:
(i) Pfropfen von n₀ Verbindungen der Formel W"₀-Gₒ-OH, wobei W"₀ eine Amin-Schutzgruppe ist, wobei n₀ und G₀ wie oben definiert sind, an eine Verbindung der Formel (II): 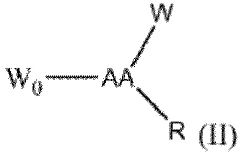 wobei R ein vorfunktionalisiertes Harz ist, W und W₀ voneinander verschieden sind und eine Amin-Schutzgruppe darstellen, wobei W von W"₀ verschieden ist, und AA wie zuvor definiert ist,
um eine Verbindung der Formel (III) zu erhalten: 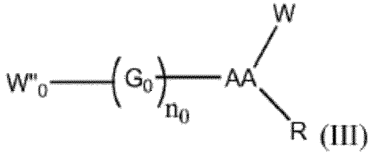
(ii) Koppeln einer Verbindung der Formel (IV): 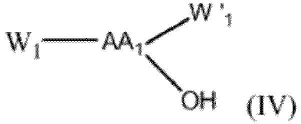 wobei W₁ und W'₁ Amin-Schutzgruppen sind, die identisch miteinander oder verschieden voneinander und identisch mit den W₀- und W"₀-Gruppen oder verschieden von diesen sind, jedoch verschieden von W sind, und AA₁ wie oben definiert ist, mit der Verbindung der Formel (III), um eine Verbindung der Formel (V) zu erhalten: 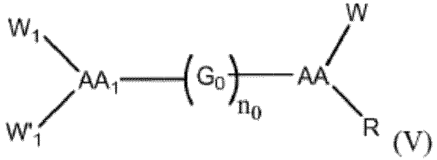
(iii) Pfropfen von 2n₁ Verbindungen der Formel W"₁-G₁-OH, wobei W"₁ eine Amin-Schutzgruppe ist, die identisch miteinander oder verschieden voneinander und identisch mit den anderen Schutzgruppen, die in diesem Verfahren verwendet werden, oder verschieden von diesen ist, jedoch verschieden von W ist, wobei G₁ und n₁ wie oben definiert sind, an die in Schritt (ii) erhaltene Verbindung, um eine Verbindung der Formel (VI) zu erhalten: 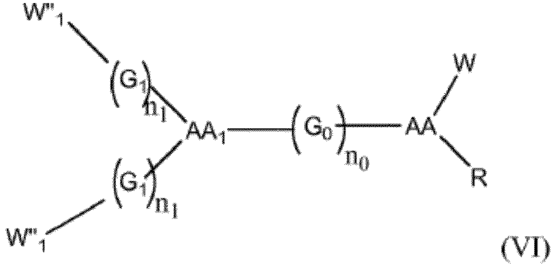
(iv) wenn X der Formel (I) Biotin ist, direktes Übergehen zu Schritt (v); andernfalls, wenn X der Formel (I) kein Biotin ist, Wiederholen der Schritte (ii) und (iii) mit 2^{p-1} Verbindungen der Formel (VII): 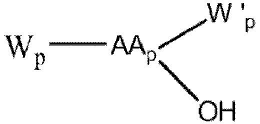 und 2^{p}nₚ-mal die Verbindung der Formel W"ₚ-Gₚ-OH, wobei p eine ganze Zahl zwischen 2 und 5 ist, und Wₚ, W'ₚ, W"ₚ Amin-Schutzgruppen sind, die identisch miteinander oder verschieden voneinander und identisch mit anderen Schutzgruppen, die in diesem Verfahren verwendet werden, oder verschieden von diesen sind, jedoch verschieden von W sind, und AA_{P} ein trifunktionelles Molekül ist, gemäß der folgenden Sequenz:
- 1-mal, wenn Y Biotin ist, wobei p dann gleich 2 ist,
- 2-mal, wenn Y nicht Biotin ist und Z Biotin ist, wobei p dann gleich 2 und ferner 3 ist,
- 3-mal, wenn Y und Z nicht Biotin sind und V Biotin ist, wobei p dann gleich 2, 3 und ferner 4 ist, und
- 4-mal, wenn Y, Z und V nicht Biotin sind, wobei p dann gleich 2, 3, 4 und ferner 5 ist,
(v) Entschützen der so erhaltenen Verbindung in Bezug auf die W"₁- oder W"ₚ-Gruppe, wobei p zwischen 2 und 5 ist, und Koppeln mit Biotin,
(vi) Entschützen, in Bezug auf die W-Gruppe, der so polybiotinylierten Verbindung und Koppeln mit einem Antiliganden oder einer reaktiven Gruppe zum Binden an einen Antiliganden (T) und
(vii) Abspalten der so erhaltenen Verbindung von dem Harz (R), um eine Verbindung der Formel (I) zu erhalten.

2. Verfahren gemäß Anspruch 1, **dadurch gekennzeichnet, dass** die Substituenten AA₁ und AA₂, gegebenenfalls AA₃, AA₄ und AA₅, identisch sind und vorzugsweise ein Lysinderivat sind.

3. Verfahren gemäß einem der Ansprüche 1 oder 2, **dadurch gekennzeichnet, dass** die Substituenten G₀, G₁, gegebenenfalls G₂, G₃, G₄ und G₅, identisch sind und vorzugsweise die Formel (-NH-CH₂-CH₂-O-CH₂-CH₂-O-CH₂-CO-) aufweisen.

4. Verfahren gemäß einem der vorhergehenden Ansprüche, **dadurch gekennzeichnet, dass** die ganzen Zahlen n₀, n₁, gegebenenfalls n₂, n₃, n₄ und n₅, identisch sind und vorzugsweise gleich 2 oder 3 sind.

5. Verfahren gemäß einem der vorhergehenden Ansprüche, **dadurch gekennzeichnet, dass** die W-Gruppe Methoxytrityl ist.

6. Verfahren gemäß einem der vorhergehenden Ansprüche, **dadurch gekennzeichnet, dass** W₁ und W'₁ identisch sind, gegebenenfalls W₂ und W'₂ identisch sind, W₃ und W'₃ identisch sind, W₄ und W'₄ identisch sind und W₅ und W'₅ identisch sind.

7. Verfahren gemäß Anspruch 6, **dadurch gekennzeichnet, dass** die Amin-Schutzgruppen W₁, W'₁ und gegebenenfalls W₂, W'₂, W₃, W'₃, W₄ und W'₄, W₅ und W'₅ Fluorenylmethoxycarbonylgruppen sind.

8. Verfahren gemäß einem der vorhergehenden Ansprüche, **dadurch gekennzeichnet, dass** der Substituent AA ein Lysinderivat ist und B NH₂ ist.

9. Verfahren gemäß einem der vorhergehenden Ansprüche, **dadurch gekennzeichnet, dass** X Folgendes ist: 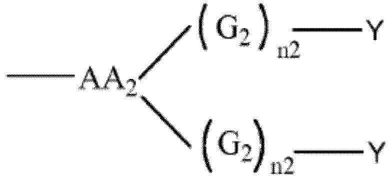 und Y Biotin ist.

10. Eine Verbindung der Formel (I): 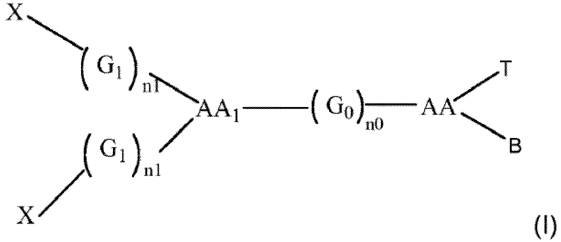 wobei
- X Biotin oder Folgendes ist: 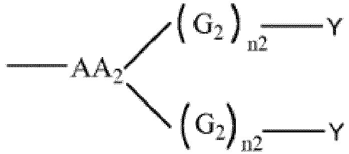
- Y Biotin oder Folgendes ist: 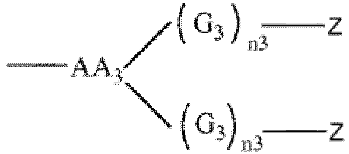
- Z Biotin oder Folgendes ist: 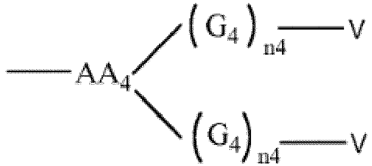
- V Biotin oder Folgendes ist: 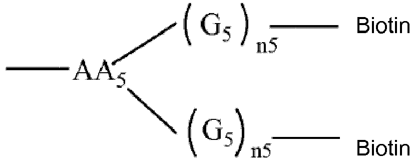
- B NH₂ oder H ist,
- AA ein Derivat eines trifunktionellen Moleküls ist, wenn B NH₂ ist, oder ein Derivat eines difunktionellen Moleküls ist, wenn B H ist,
- AA₁ bis AA₄ jeweils unabhängig ein Derivat eines trifunktionellen Moleküls sind,
- G₀ bis G₅ jeweils unabhängig ein Arm, beinhaltend eine (-CH₂-CH₂-O-)-Einheit, sind,
- n₀ bis n₅ jeweils unabhängig eine ganze Zahl von 1 bis 8 sind und
- T eine Maleimidgruppe, eine Carbonsäuregruppe oder ein Antiligand, der aus Hapten, Antikörper, Antigen, Peptid, Zucker, Lektin und Polynukleotid ausgewählt ist, ist; wobei das trifunktionelle Molekül drei identische oder verschiedene funktionelle Gruppen, die aus NH₂ und COOH ausgewählt sind, umfasst.

11. Verbindung der Formel (I) gemäß Anspruch 10, **dadurch gekennzeichnet, dass** die Substituenten AA₁ und AA₂, gegebenenfalls AA₃, AA₄ und AA₅, identisch sind und vorzugsweise ein Lysinderivat sind.

12. Verbindung der Formel (I) gemäß einem der Ansprüche 10 und 11, **dadurch gekennzeichnet, dass** die Substituenten G₀, G₁, gegebenenfalls G₂, G₃, G₄ und G₅, identisch sind und vorzugsweise die Formel (-NH-CH₂-CH₂-O-CH₂-CH₂-O-CH₂-CO-) aufweisen.

13. Verbindung der Formel (I) gemäß einem der Ansprüche 10 bis 12, **dadurch gekennzeichnet, dass** die ganzen Zahlen n₀, n₁, gegebenenfalls n₂, n₃, n₄ und n₅, identisch sind und vorzugsweise gleich 2 oder 3 sind.

14. Verbindung der Formel (I) gemäß einem der Ansprüche 10 bis 13, **dadurch gekennzeichnet, dass** X Folgendes ist: 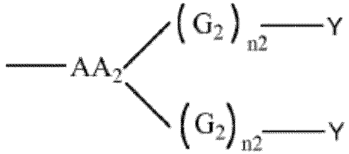 und Y Biotin ist.

15. Verbindung der Formel (I) gemäß einem der Ansprüche 10 bis 14, **dadurch gekennzeichnet, dass** AA ein Lysinderivat ist und B NH₂ ist.

16. Verbindung der Formel (I) gemäß einem der Ansprüche 10 bis 15, **dadurch gekennzeichnet, dass** der Antiligand ein Fab'-Fragment ist.

17. Verbindung der Formel (I) gemäß Anspruch 10, **dadurch gekennzeichnet, dass**:
- X Folgendes ist: 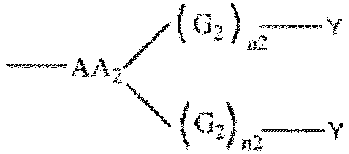
- Y Biotin ist,
- AA, AA₁ und AA₂ ein Lysinderivat sind,
- G₀, G₁ und G₂ die Formel -(NH-CH₂-CH₂-O-CH₂-CH₂-O-CH₂-CO)- aufweisen,
- n₀, n₁ und n₂ gleich 2 sind,
- B NH₂ ist und
- T ein Antiligand, vorzugsweise ein Fab'-Fragment, ist.

18. Eine Verwendung der nach dem Verfahren gemäß den Ansprüchen 1 bis 9 erhaltenen Verbindungen zur Signalverstärkung in einem diagnostischen Test.

19. Eine Verwendung der in den Ansprüchen 10 bis 17 beschriebenen Verbindungen zur Signalverstärkung in einem diagnostischen Test.

## Claims

1. A method of preparing a compound of formula (I): 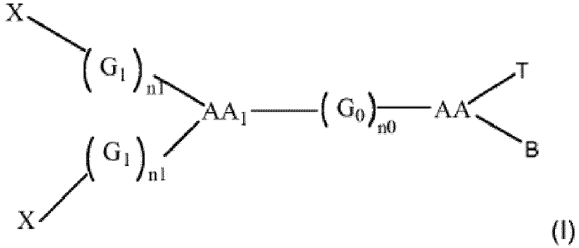 wherein
- X is biotin or 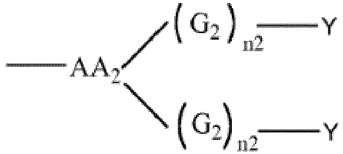
- Y is biotin or 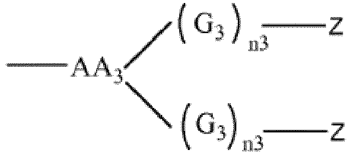
- Z is biotin or 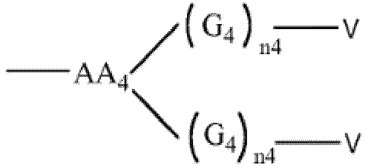
- V is biotin or 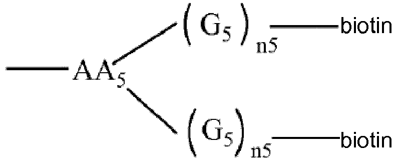
- B is NH₂ or H,
- AA is a trifunctional molecule derivative when B is NH₂ or a bifunctional molecule derivative when B is H,
- AA₁ to AA₅ are each independently a trifunctional molecule derivative,
- G₀ to G₅ are each independently an arm comprising at least one (-CH₂-CH₂-O) unit,
- n₀ to n₅ are each independently a whole number between 1 and 8 and
- T is an antiligand chosen from: hapten, antibody, antigen, peptide, sugar, lectin and polynucleotide or a reactive group for binding to an antiligand, said antiligand being capable of reacting with a ligand,
**characterised in that** it comprises the steps consisting of:
(i) grafting n₀ compounds of formula W"₀-G₀-OH, wherein W"₀ is an amine-protecting group, n₀ and G₀ being such as defined above, to a compound of formula (II): 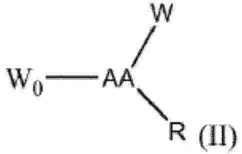 wherein R is a prefunctionalised resin, W and W₀ are different from one another and represent an amine-protecting group, W being different from W"o, and AA is such as defined above,
to obtain a compound of formula (III): 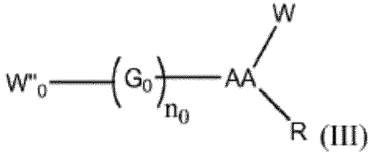
(ii) coupling a compound of formula (IV): 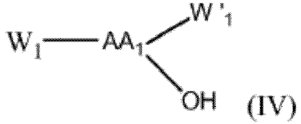 wherein W₁ and W'₁ are amine-protecting groups that are identical to or different from one another and identical to or different from W₀ and W"₀ groups, but different from W, and AA₁ is such as defined above, with the compound of formula (III) to obtain a compound of formula (V): 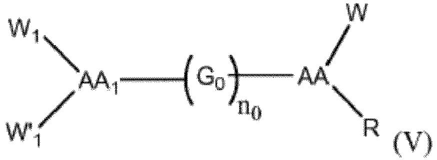
(iii) grafting 2n₁ compounds of formula W₁-G₁-OH, wherein W"₁ is an amine-protecting group, identical to or different from one another and identical to or different from other protecting groups used in this method, but different from W, G₁ and n₁ being such as defined above, onto the compound obtained in step (ii) to obtain a compound of formula (VI): 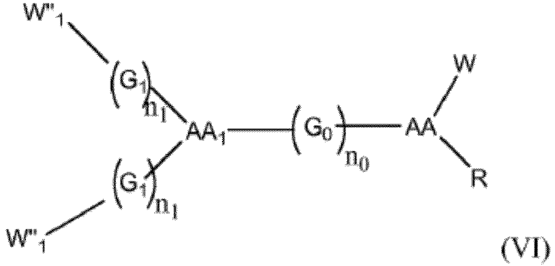
(iv) when X in formula (I) is biotin, proceeding directly to step (v); otherwise, when X in formula (I) is not biotin, repeating steps (ii) and (iii) with 2^{P-1} compounds of formula (VII): 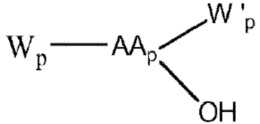 and 2^{P}nₚ times the compound of formula W"ₚ-Gₚ-OH, wherein p is a whole number between 2 and 5, and Wₚ, W'ₚ, W"ₚ are amine-protecting groups that are identical to or different from one another and identical to or different from other protecting groups used in this method, but different from W, and AAₚ is a trifunctional molecule, according to the following sequence:
- 1 time when Y is biotin, p then being equal to 2
- 2 times when Y is not biotin and Z is biotin, p then being equal to 2 then to 3,
- 3 times when Y and Z are not biotin and V is biotin, p then being equal to 2, 3 then 4, and
- 4 times when Y, Z and V are not biotin, p then being equal to 2, 3, 4 then 5,
(v) deprotecting the compound thus obtained on the W"₁ or W"ₚ group, p being between 2 and 5, and coupling with biotin,
(vi) deprotecting, on the W group, the compound thus polybiotinylated and coupling with an antiligand or a reactive group for binding to an antiligand (T) and
(vii) cutting the compound thus obtained from the resin (R) to obtain a compound of formula (I).

2. The method according to claim 1, **characterised in that** the substituents AA₁ and AA₂, if applicable AA₃, AA₄ and AA₅, are identical and are preferably a lysine derivative.

3. The method according to any one of claims 1 or 2, **characterised in that** the substituents G₀, G₁, if applicable G₂, G₃, G₄ and G₅ are identical and are preferably of formula (-NH-CH₂-CH₂-O-CH₂-CH₂-O-CH₂-CO-).

4. The method according to one of the preceding claims, **characterised in that** the whole numbers n₀, n₁, if applicable n₂, n₃, n₄ and n₅, are identical and are preferably equal to 2 or 3.

5. The method according to any one of the preceding claims, **characterised in that** the W group is methoxytrityl.

6. The method according to any one of the preceding claims, **characterised in that** W₁ and W'₁ are identical and, if applicable, W₂ and W'₂ are identical, W₃ and W'₃ are identical, W₄ and W'₄ are identical and W₅ and W'₅ are identical.

7. The method according to claim 6, **characterised in that** the amine-protecting groups W₁, W'₁ and, if applicable, W₂, W'₂, W₃, W'₃, W₄ and W'₄, W₅ and W'₅ are fluorenylmethoxycarbonyl groups.

8. The method according to any one of the preceding claims, **characterised in that** the substituent AA is a lysine derivative and B is NH₂.

9. The method according to any one of the preceding claims, **characterised in that** X is 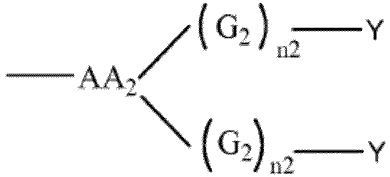 and Y is biotin.

10. A compound of formula (I): 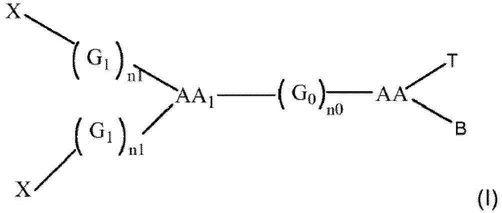 wherein
- X is biotin or 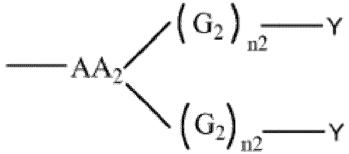
- Y is biotin or 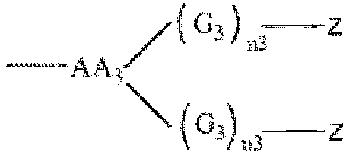
- Z is biotin or 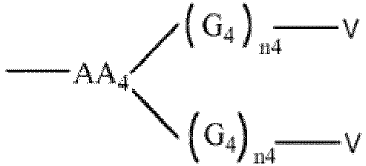
- V is biotin or 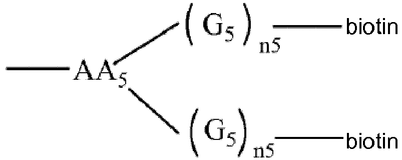
- B is NH₂ or H,
- AA is a trifunctional molecule derivative when B is NH₂ or a bifunctional molecule derivative when B is H,
- AA₁ to AA₄ are each independently a trifunctional molecule derivative,
- G₀ to G₅ are each independently an arm comprising a (-CH₂-CH₂-O-) unit,
- n₀ to n₅ are each independently a whole number between 1 and 8 and
- T is a maleimide group, a carboxylic acid group or an antiligand chosen from: hapten, antibody, antigen, peptide, sugar, lectin and polynucleotide; said trifunctional molecule including three identical or different functions chosen from NH₂ and COOH.

11. The compound of formula (I) according to claim 10, **characterised in that** the substituents AA₁ and AA₂, if applicable AA₃, AA₄ and AA₅, are identical and are preferably a lysine derivative.

12. The compound of formula (I) according to any one of claims 10 and 11, **characterised in that** the substituents G₀, G₁, if applicable G₂, G₃, G₄ and G₅, are identical and are preferably of formula (-NH-CH₂-CH₂-O-CH₂-CH₂-O-CH₂-CO-).

13. The compound of formula (I) according to any one of claims 10 to 12, **characterised in that** the whole numbers n₀, n₁, if applicable n₂, n₃, n₄ and n₅, are identical and are preferably equal to 2 or 3.

14. The compound of formula (I) according to any one of claims 10 to 13, **characterised in that** X is 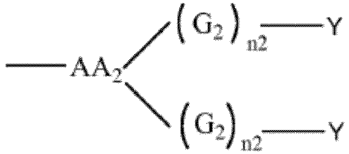 and Y is biotin.

15. The compound of formula (I) according to any one of claims 10 to 14, **characterised in that** AA is a lysine derivative and B is NH₂.

16. The compound of formula (I) according to any one of claims 10 to 15, **characterised in that** the antiligand is a Fab' fragment.

17. The compound of formula (I) according to claim 10, **characterised in that**:
- X is 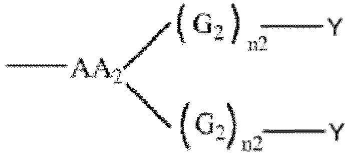
- Y is biotin,
- AA, AA₁ and AA₂ are a lysine derivative,
- G₀, G₁ and G₂ are of formula -(NH-CH₂-CH₂-O-CH₂-CH₂-O-CH₂-CO)-,
- n₀, n₁ and n₂ are equal to 2,
- B is NH₂ and
- T is an antiligand, preferably a Fab' fragment.

18. Use of the compounds obtained by the method as described in claims 1 to 9 for signal amplification in a diagnostic test.

19. Use of the compounds as described in claims 10 to 17 for signal amplification in a diagnostic test.
